# EUROPEAN PATENT SPECIFICATION

(11) **EP 4 468 999 B1**
(45) Date of publication and mention of the grant of the patent: **09.12.2026**
(21) Application number: 23702904.6
(22) Date of filing: 20.01.2023
(51) Int. Cl.: A61F 2/24, A61F 2/95

(54) **SYSTEMS FOR TRANSCATHETER VALVE REPLACEMENT AND METHODS**
SYSTEME FÜR TRANSKATHETERKLAPPENERSATZ UND VERFAHREN
SYSTÈMES DE REMPLACEMENT DE VALVULE TRANSCATHÉTER ET PROCÉDÉS

(30) Priority: 28.01.2022 US 202263304026 P
(43) Date of publication of application: 04.12.2024
(73) Proprietor: Medtronic, Inc., Minneapolis, Minnesota 55432 (US)
(72) Inventor: HAINES, Michael, Richmond, Victoria 3121 (AU); WIEMEYER, Nathan, Santa Rosa, California 95403 (US); WISMAN, Elise, Santa Rosa, California 95403 (US); CHEN, Claire, Santa Rosa, California 95403 (US); MAUCH, Kevin, Santa Rosa, California 95403 (US); CACERES, Alejandra, Santa Rosa, California 95403 (US); ZAVER, Nrupen, Santa Rosa, California 95403 (US); NOVAK, Mikayla, Santa Rosa, California 95403 (US)
(74) Representative: Zimmermann & Partner Patentanwälte mbB
(86) International application number: PCT/IB2023/050496
(87) International publication number: WO 2023/144671

(56) References cited:
- WO-A1-2019/014473
- US-A1- 2018 256 326
- US-A1- 2019 008 636

## Description

### FIELD

The present technology is generally related to systems and methods for transcatheter delivery and deployment of an implant or prosthesis to an annulus, such as a heart valve.

### BACKGROUND

A human heart includes four heart valves that determine the pathway of blood flow through the heart: the mitral valve, the tricuspid valve, the aortic valve, and the pulmonary valve. The mitral and tricuspid valves are atrio-ventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Diseased or otherwise deficient heart valves can be repaired or replaced using a variety of different types of heart valve surgeries. One conventional technique involves an open-heart surgical approach that is conducted under general anesthesia, during which the heart is stopped and blood flow is controlled by a heart-lung bypass machine.

More recently, minimally invasive approaches have been developed to facilitate catheter-based implantation of a prosthetic heart valve or prosthesis on the beating heart, intending to obviate the need for the use of classical sternotomy and cardiopulmonary bypass. In general terms, an expandable prosthetic valve is compressed about or within a catheter, inserted inside a body lumen of the patient, such as the femoral artery, and delivered to a desired location in the heart.

The heart valve prosthesis employed with catheter-based, or transcatheter, procedures generally includes an expandable multi-level frame or stent that supports a valve structure having a plurality of leaflets. The frame can be contracted during percutaneous transluminal delivery, and expanded upon deployment at or within the native valve. One type of valve stent can be initially provided in an expanded or un-crimped condition, then crimped or compressed about a balloon portion of a catheter. The balloon is subsequently inflated to expand and deploy the prosthetic heart valve. With other stented prosthetic heart valve designs, the stent frame is formed to be self-expanding. With these systems, the valved stent is crimped down to a desired size and held in that compressed state within a sheath for transluminal delivery. Retracting the sheath from this valved stent allows the stent to self-expand to a larger diameter, fixating at the native valve site. In more general terms, then, once the prosthetic valve is positioned at the treatment site, for instance within an incompetent native valve, the stent frame structure may be expanded to hold the prosthetic valve firmly in place.

The present disclosure addresses problems and limitations associated with the related art.

WO2019/014473A1 * relates to prosthetic heart valves and apparatus and methods for delivery of same. US 2018/0256326 A1 relates to delivery systems having a short capsule segment and a cinch mechanism, US 2019/0008636 A1 relates to prosthetic heart valve devices and associated systems and methods.

### SUMMARY

The techniques of this disclosure generally relate to systems and methods for transcatheter delivery and deployment of an implant or prosthesis, such as a prosthetic heart valve, to a target site. Aspects of the disclosure are particularly beneficial for transcatheter tricuspid replacement as various delivery systems are configured to reduce the depth in which the device needs to be inserted into the right ventricle during delivery of the prosthetic heart valve. Access to a tricuspid valve can be challenging in that existing implanted devices may be in the anatomy, reducing the space available for the delivery system. In addition, visualization of the delivery system and implant may be challenging as metallic delivery system components can cause artifacts due to density. Further, chordae and papillary muscles serve as obstacles for delivery and the right ventricle is generally shorter than the left ventricle. All of these considerations result in a general desire for a system capable of delivering an implant to a tricuspid valve while reducing a length the delivery system extends into the right ventricle and past the valve annulus to increase the population of patients that are candidates for the systems and methods of the disclosure. It will be understood that such systems and methods can also be utilized for other procedures, such as mitral valve replacement, for example.

The invention is defined by the appended claims.

According to a first embodiment hereof, the present disclosure provides a prosthesis delivery system including a tubular capsule and a prosthesis. The prosthesis includes a brim having a first end. The prosthesis delivery system has a delivery configuration where a portion of the prosthesis is disposed in the capsule and at least a portion of the brim is outside the capsule and everted to extend toward the capsule prior to deployment of the prosthesis from the capsule.

In an aspect of the first embodiment, the disclosure provides that the tubular capsule has a closed end and an open end.

In an aspect of the first embodiment, the disclosure provides that the first end of the brim is releasably coupled to the capsule.

In an aspect of the first embodiment, the disclosure provides that the prosthesis delivery system has a partially deployed configuration where the brim has been released from the capsule.

In an aspect of the first embodiment, the disclosure provides that the brim is configured to move toward a reverted position when released.

In an aspect of the first embodiment, the disclosure provides that the first end of the brim is tucked inside the capsule.

In an aspect of the first embodiment, the disclosure provides that the capsule has an open end with a raised lip. The first end of the brim is tucked in the raised lip.

In an aspect of the first embodiment, the disclosure provides that the brim is releasably coupled to the capsule with sutures.

In an aspect of the first embodiment, the disclosure provides that the prosthesis has a plurality of fixation elements. The everted brim covers the plurality of fixation elements.

In an aspect of the first embodiment, the disclosure provides that the prosthesis is a self-expanding prosthesis configured to expand from a compressed configuration toward an expanded configuration.

In an aspect of the first embodiment, the disclosure provides that the prosthesis is a prosthetic heart valve.

In an aspect of the embodiment, the disclosure provides that the tubular capsule has a closed end and an open end. The delivery system further includes a piston positioned within the capsule. The closed end and the piston form at least in part a chamber and a fluid source is coupled to the chamber.

In an aspect of the embodiment, the disclosure provides that the prosthesis includes a frame, and the frame is coupled to the brim.

In an aspect of the first embodiment, the disclosure provides that the brim extends from the frame.

In an aspect of the first embodiment, the disclosure provides that at least a portion of the frame extends out from the capsule.

In an aspect of the embodiment, the disclosure provides that the brim and the capsule collectively entirely cover the frame.

In an aspect of the embodiment, the disclosure provides that the brim extends from the frame.

In an aspect of the first embodiment, the disclosure provides that the capsule is configured to distally advance to unsheathe the prosthesis.

According to a second embodiment hereof, the present disclosure provides a method of delivering a prosthesis. A system is provided in a delivery configuration. The system includes a delivery system having a tubular capsule and a prosthesis loaded partially within the tubular capsule in a delivery configuration. The prosthesis includes an anchoring member and a brim connected to the anchoring member. In the delivery configuration, the prosthesis is at least partially disposed within the capsule and at least a portion of the brim is outside of the capsule and is everted to extend toward the capsule prior to deployment of the prosthesis from the capsule. The capsule is directed to a target site in the delivery configuration. The capsule is distally advanced, allowing the brim to at least partially revert.

In an aspect of the second embodiment, the disclosure provides that the target site is a heart valve.

In an aspect of the second embodiment, the disclosure provides that the method further includes the step of unlocking the brim to at least partially revert the brim.

In an aspect of the second embodiment, the disclosure provides that a proximal end of the capsule includes a raised lip that engages the brim in the delivery configuration.

In an aspect of the second embodiment, the disclosure provides that the brim is tucked into a proximal end of the capsule in the delivery configuration.

In an aspect of the second embodiment, the disclosure provides that the brim includes a flexible web and a brim support attached to the flexible web.

In an aspect of the second embodiment, the disclosure provides that the brim support is positioned within the capsule in the delivery configuration.

In an aspect of the second embodiment, the disclosure provides that the capsule is directed via transcatheter procedure through an inferior vena cava into a right atrium and the capsule is further directed down though a tricuspid valve annulus and at least partially within a right ventricle.

According to a third embodiment, the present disclosure provides a method of loading a prosthesis into a delivery capsule. A prosthesis having a support structure and a brim extending from the support structure is positioned into a tubular capsule such that a portion of the support structure extends from the capsule. The brim is everted such that the brim and the tubular capsule cover the support structure.

In an aspect of the third embodiment, the disclosure provides that the prosthesis is everted such that the prosthesis and capsule completely cover the prosthesis support structure.

In an aspect of the third embodiment, the disclosure provides that the method further includes the step of tucking a portion of the prosthesis into the capsule.

In an aspect of the third embodiment, the disclosure provides that a valve is supported in the prosthesis support structure.

In an aspect of the third embodiment, the disclosure provides that the support structure comprises a frame.

In an aspect of the third embodiment, the disclosure provides that the support structure comprises an inner frame and an outer frame with the inner frame positioned in the outer frame.

According to a fourth embodiment, the present disclosure provides a method of loading a prosthesis into a delivery capsule. A prosthesis having a support structure and a brim extending from the support structure is positioned into a tubular capsule such that a portion of the support structure extends from the capsule. The portion of the support structure that extends from the capsule is covered with the brim.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 depicts a perspective view of a prosthetic heart valve in accordance with an aspect of the disclosure.
FIG. 2 depicts a perspective view of a valve support of the prosthetic heart valve of FIG. 1 with a valve component secured therein in accordance with an aspect of the disclosure.
FIG. 3 depicts an atrial end view of the prosthetic heart valve shown in FIG. 1 in accordance with an aspect of the disclosure.
FIG. 4 depicts a ventricular end view of the prosthetic heart valve shown in FIG. 1 in accordance with an aspect of the disclosure.
FIG. 5 depicts a side view of a delivery system according to an embodiment hereof, wherein the delivery system is configured for delivering the prosthetic heart valve of FIG. 1 within a capsule of the delivery system.
FIG. 5A is a cross-sectional view taken along line A-A of FIG. 5.
FIG. 5B is a sectional view of the delivery system of FIG. 5.
FIG. 6 is an exploded view of the delivery system of FIG. 5.
FIG. 6A is an enlarged view of a distal portion of an innermost shaft assembly of the delivery system of FIG. 6, wherein the innermost shaft assembly includes a flexible shaft, a piston mount, a piston, a tension cable, a distal shaft, a capsule, and a capsule cap.
FIG. 6B is an enlarged view of a first subassembly of the innermost shaft assembly of FIG. 6A, wherein the first subassembly includes the flexible shaft, the piston mount and the piston.
FIG. 6C is an enlarged view of a second subassembly of the innermost shaft assembly of FIG. 6A, wherein the second subassembly includes the tension cable, the distal shaft, the capsule, and the capsule cap.
FIG. 6D is a sectional view of the distal shaft and the capsule cap of FIG. 6C.
FIG. 7 is a perspective view of a piston of the delivery system of FIG. 5, wherein the piston is shown removed from the delivery system for sake of illustration only.
FIG. 8 is a side view of a distal portion of the delivery system of FIG. 5, wherein the distal portion includes the capsule and the piston, the capsule being shown in a first position relative to the piston. The prosthetic heart valve of FIG. 1 is loaded into the capsule of the delivery system and a brim of the prosthetic heart valve of FIG. 1 is not yet everted.
FIG. 9 is a side view of the distal portion of FIG. 7, the capsule being shown in a second position relative to the piston. The prosthetic heart valve of FIG. 1 is not shown for sake of illustration only.
FIG. 10 is a side view of the distal portion of FIG. 7, the capsule being shown in a third position relative to the piston. The prosthetic heart valve of FIG. 1 is not shown for sake of illustration only.
FIG. 11 is another side view of the distal portion of FIG. 7, wherein the prosthetic heart valve of FIG. 1 is initially loaded into the capsule of the delivery system for delivery thereon and the brim of the prosthetic heart valve of FIG. 1 is not yet everted.
FIG. 11A is a side view of a distal portion of a delivery system including a relatively shorter capsule, according to another embodiment hereof.
FIG. 12 is a side view of the distal portion of FIG. 7, wherein the prosthetic heart valve of FIG. 1 is loaded into the capsule of the delivery system for delivery thereon and the prosthetic heart valve of FIG. 1 is in a delivery configuration with the brim thereof everted.
FIG. 13A is a sectional view of FIG. 12, with only the capsule and the prosthetic heart valve of FIG. 1 shown for sake of illustration.
FIG. 13B is a sectional view according to another embodiment hereof, with only the capsule and the prosthetic heart valve of FIG. 1 shown for sake of illustration.
FIG. 13C is a sectional view according to another embodiment hereof, with only the capsule and the prosthetic heart valve of FIG. 1 shown for sake of illustration.
FIG. 14 is a sectional view according to another embodiment hereof, with only a capsule and the prosthetic heart valve of FIG. 1 shown for sake of illustration, wherein the capsule includes a raised lip configured to tucking the brim of the prosthetic heart valve thereunder.
FIG. 15 is an enlarged sectional view of the raised lip of the capsule of FIG. 14.
FIG. 16 is a schematic top view of a brim for a prosthetic heart valve according to another embodiment hereof, wherein the brim includes a plurality of echogenic ribs, each echogenic rib attached to a surface of the brim that is exterior when everted.
FIG. 17 is a schematic top view of a brim for a prosthetic heart valve according to another embodiment hereof, wherein the brim includes a plurality of echogenic ribs, each echogenic rib attached to a surface of the brim that is exterior when everted, and wherein each echogenic rib extends from a first end to a second end of the brim.
FIG. 18 is a schematic view of a brim for a prosthetic heart valve according to another embodiment hereof, wherein the brim includes a single layer of material and a plurality of exposed peaks that are not covered by the single layer of material.
FIG. 19 schematically illustrates one example method of the disclosure.
FIG. 20A is a perspective view of the delivery system of FIG. 5, wherein the prosthetic heart valve of FIG. 1 is loaded into the capsule of the delivery system for delivery thereon and the prosthetic heart valve of FIG. 1 is in a delivery configuration with the brim thereof everted.
FIGS. 20B-20C are perspective views of the system of FIG. 20A in which the brim is in a partially-reverted position or arrangement.
FIGS. 20D-20E are perspective views of the system of FIGS. 20A-20C in which the brim is in a fully reverted position or arrangement and at least part of an anchoring member of the prosthetic heart valve is freed from the capsule so that it self-expands.
FIGS. 21A-21D are side views of the delivery system of FIG. 5 having been modified to allow for recapture or recompression of the prosthetic heart valve after partial deployment.
FIG. 22 depicts a perspective view of a prosthetic heart valve in accordance with an aspect of the disclosure, wherein a connection element of the prosthetic heart valve attaches an inner frame to an outer frame thereof and the connection element is further configured to releasably couple to the piston of the delivery system of FIG. 5.
FIG. 23 is an enlarged schematic view of a connection element of the prosthetic heart valve of FIG. 1.
FIG. 24 is an enlarged schematic view of the connection element of the prosthetic heart valve of FIG. 22.
FIG. 25 depicts a perspective view of a prosthetic heart valve in accordance with another embodiment hereof.

### DETAILED DESCRIPTION

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician. With respect to a prosthetic valve device, the terms "proximal" and "distal" can refer to the location of portions of the device with respect to the direction of blood flow. For example, proximal can refer to an upstream position or a location where blood flows into the device (e.g., inflow region), and distal can refer to a downstream position or a location where blood flows out of the device (e.g., outflow region).

As referred to herein, implants, prostheses, prosthetic heart valves or prosthetic valves useful with the various systems, devices and methods of the present disclosure may assume a wide variety of configurations. Prosthetic heart valves can include, for example, a bioprosthetic heart valve having tissue leaflets or a synthetic heart valve having polymeric, metallic or tissue-engineered leaflets, and can be specifically configured for replacing valves of the human heart. The prosthetic valves of the present disclosure may be self-expandable, balloon expandable and/or mechanically expandable or combinations thereof. In general terms, the prosthetic valves of the present disclosure include a stent or stent frame having an internal lumen maintaining a valve structure (tissue or synthetic), with the stent frame having a normal, expanded condition or arrangement and collapsible to a compressed condition or arrangement for loading within the delivery system. For example, the stents or stent frames are support structures that comprise a number of struts or wire segments arranged relative to each other to provide a desired compressibility and strength to the prosthetic valve. The struts or wire segments are arranged such that they are capable of self-transitioning from, or being forced from, a compressed or collapsed arrangement to a normal, radially expanded arrangement. The struts or wire segments can be formed from a shape memory material, such as a nickel titanium alloy (e.g., Nitinol). The stent frame can be laser-cut from a single piece of material, or can be assembled from a number of discrete components.

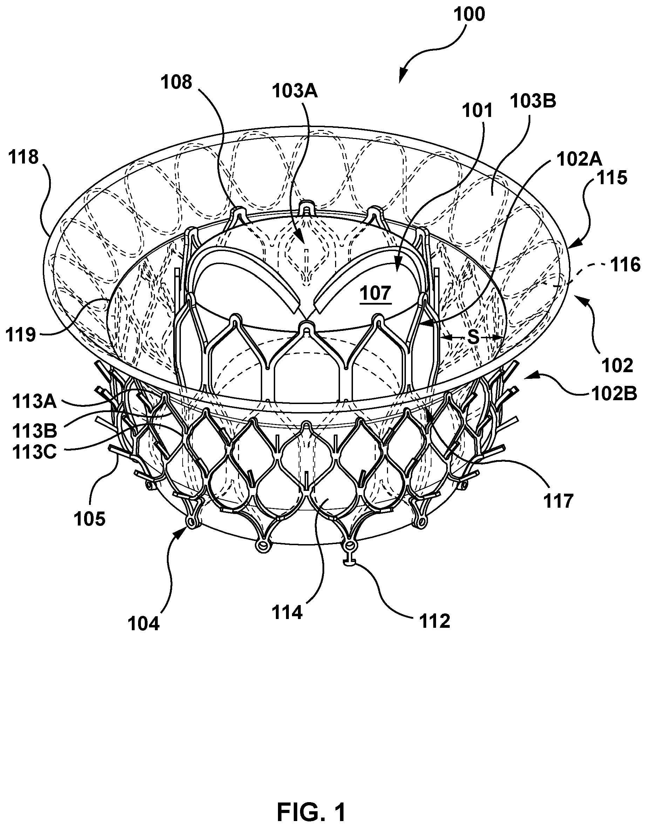

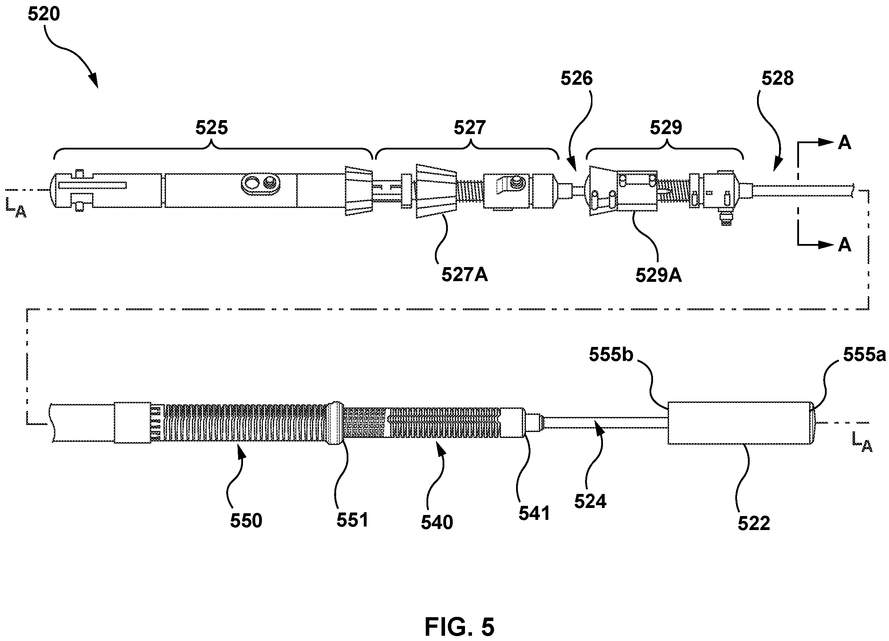

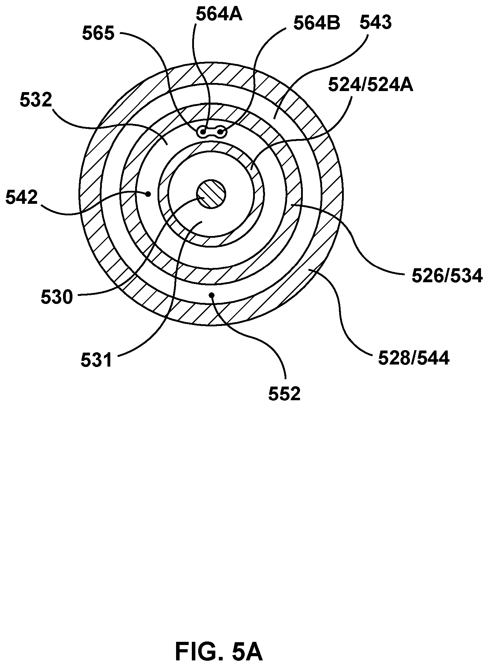

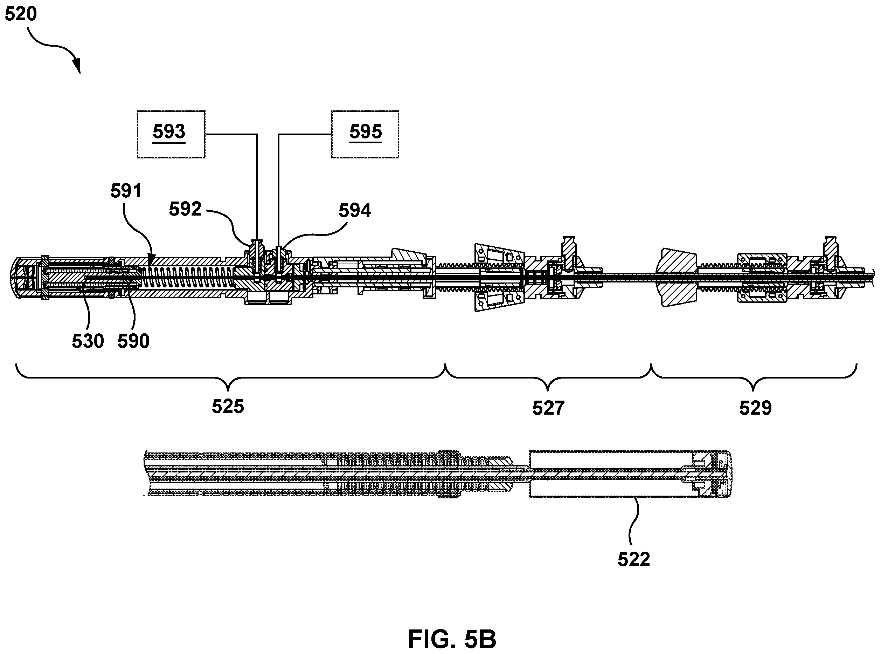

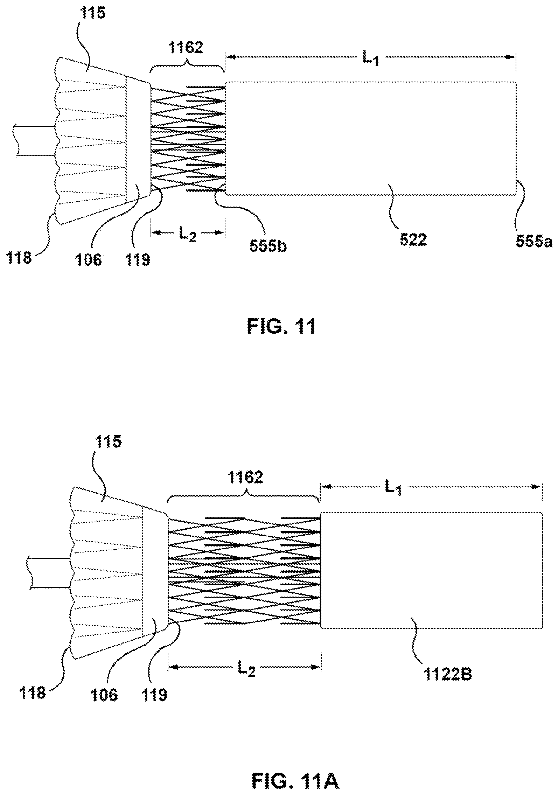

Systems and methods of the disclosure include a delivery system having a capsule for radially compressing a prosthetic heart valve. In embodiments hereof, the capsule is shorter than the prosthetic heart valve and does not cover the entire length thereof during delivery within a vasculature. A brim of the prosthetic heart valve is configured to distally fold or evert so that the everted brim and the capsule collectively sheathe the entire length of the prosthetic heart valve during delivery of the prosthetic heart valve to the target site. The detailed description hereof first includes a description of an exemplary prosthetic heart valve in FIGS. 1-4 that may be used in embodiments hereof, and further includes a description of a delivery system in FIGS. 5-10 that may be used in embodiments hereof. The interaction between the prosthetic heart valve and the delivery system, in which the brim of the prosthetic heart valve everts to cover a portion of the prosthetic heart valve during delivery according to various embodiments hereof, is further described in FIGS. 11-20E. It will be understood by one of ordinary skill in the art that the prosthetic heart valve of FIGS. 1-4 and the delivery system of FIGS. 5-10 are exemplary, and alternative configurations thereof may be utilized in accordance with the principles described herein.

FIGS. 1-4 illustrate an exemplary prosthetic heart valve 100 for use in embodiments hereof. Prosthetic heart valve 100 is illustrated herein in order to facilitate description of the interaction between the prosthetic heart valve 100 and a delivery system to be utilized in conjunction therewith according to embodiments hereof. It is understood that any number of alternate heart valve prostheses can be used with the methods and devices described herein. The prosthetic heart valve 100 is presented by way of example only, and other shapes and designs of prosthetic heart valves are also consistent with embodiments hereof. Other non-limiting examples of prosthetic heart valves that can be delivered via the delivery systems and methods described herein are described in U.S. Appl. No. 16/853,851 to McVeigh et al.*,* U.S. Patent No. 9,034,032 to McLean et al. and International Patent Application No. PCT/US5114/029549 to McLean et al. Although the prosthetic heart valve 100 is configured for placement within a tricuspid heart valve or a mitral heart valve, embodiments of delivery systems and techniques described herein may be used in conjunction with any transcatheter valve prostheses. For example, embodiments described herein may be utilized with a transcatheter prosthetic heart valve configured for placement within a pulmonary, aortic, mitral, or tricuspid valve. There is no intention to be bound by any expressed or implied theory presented in the preceding technical field, background, brief summary or the following detailed description.

The prosthetic heart valve 100 is configured to be radially compressed into a reduced-diameter configuration (not shown) for delivery within a vasculature and to return to an expanded, deployed configuration, which is shown in FIGS. 1-4. Stated another way, the prosthetic heart valve 100 has a crimped configuration for delivery within a vasculature and an expanded configuration for deployment within a native heart valve. In accordance with embodiments hereof, when in the radially compressed or reduced-diameter configuration, the prosthetic heart valve 100 has a low profile suitable for delivery to and deployment within a native heart valve via a suitable delivery system that may be tracked to the deployment site of the native heart valve of a heart via any one of a transatrial, antegrade, or transapical approach. The prosthetic heart valve 100 includes a stent or frame 102 and a valve component 101 including at least one leaflet 107 disposed within and secured to the frame 102. The valve component 101 of the prosthetic heart valve 100 is capable of regulating flow therethrough via valve leaflets that may form a replacement valve.

Any portion of the frame 102 described herein as an element of a heart valve prothesis 100 may be made from any number of suitable biocompatible materials, e.g., stainless steel, nickel titanium alloys such as Nitinol^{™}, cobalt chromium alloys such as MP35N, other alloys such as ELGILOY^{®} (Elgin, Ill.), various polymers, pyrolytic carbon, silicone, polytetrafluoroethylene (PTFE), or any number of other materials or combination of materials. A suitable biocompatible material would be selected to provide the transcatheter heart valve prothesis 100 to be configured to be compressed into a reduced-diameter crimped configuration for transcatheter delivery to a native valve, whereby release from a delivery catheter returns the prosthesis to an expanded, deployed configuration. Alternatively, the prosthetic heart valve 100 may be balloon-expandable as would be understood by one of ordinary skill in the art.

In an aspect of the disclosure, the frame 102 of the prosthetic heart valve 100 includes a valve support 102A at least partially surrounded by and attached to an anchoring member 102B. The valve support 102A is configured to support the valve component 101 therein. The valve support 102A is a tubular stent-like or frame structure that defines a central lumen from a first end 108 of the valve support 102A to a second end 109 of the valve support 102A. When positioned *in situ* within a native triscupid valve, the first end 108 is an inflow or upstream end and the second end 109 is an outflow or downstream end. At the outflow end 109, the valve support 102A is attached to the anchoring member 102B via a plurality of connector components 104. In an embodiment, the plurality of connector components are rivets.

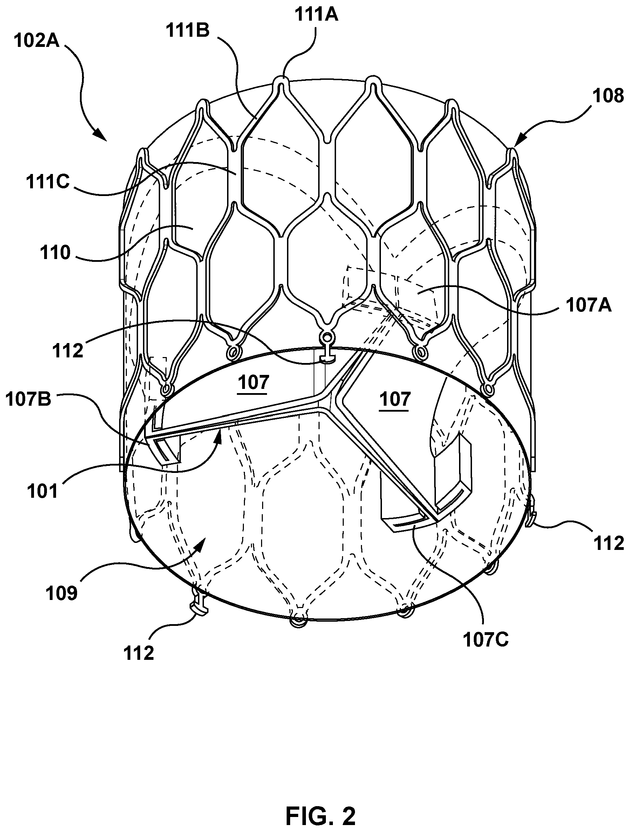

Referring to FIG. 2, the structure of the valve support 102A will now be described in more detail. The valve support 102A includes a plurality of crowns 111A and a plurality of struts 111B with each crown 111A being formed between a pair of opposing struts 111B. Each crown 111A is a curved segment or bend extending between opposing struts 111B. The valve support 102A is tubular, with a plurality of side openings 110 being defined by edges of the plurality of crowns 111A and the plurality of struts 111B. In an embodiment, the plurality of side openings 110 may be substantially diamond-shaped. The valve support 102A includes a plurality of nodes 111C. A node 111C is defined as a region where two crowns of the plurality of crowns 111A within the valve support 102A meet or connect. At the outflow end 109 thereof, the valve support 102A includes a plurality of attachment bars 112 extending therefrom that function to releasably couple the prosthetic heart valve 100 to a delivery system. In an embodiment, the valve support 102A includes exactly three attachment bars 112 that are circumferentially spaced apart from each other at equal intervals.

The anchoring member 102B is a stent-like or frame structure that functions as an anchor for the prosthetic heart valve 100 to secure its deployed position within a native annulus. The anchoring member 102B is a substantially cylindrically-shaped structure that is configured to engage heart tissue at or below an annulus of a native heart valve, such as an annulus of a native mitral valve. At the inflow end 108 of the valve support 102A, the anchoring member 102B is radially spaced a distance S from the valve support 102A to mechanically isolate the inflow end 108 of the valve support 102A from the anchoring member 102B. The anchoring member 102B includes one or more fixation elements 105 that extend outward from an exterior side thereof to engage heart tissue. The fixation elements 105 project radially outward and are inclined toward an upstream direction. The fixation elements 105, for example, can be prongs, cleats, barbs, hooks, or other elements that are inclined only in the upstream direction (e.g., a direction extending away from the downstream portion of the prosthetic heart valve 100. In an embodiment, the anchoring member 102 includes exactly three rows of fixation elements 105.

The anchoring member 102B includes a plurality of crowns 113A and a plurality of struts 113B with each crown 113A being formed between a pair of opposing struts 113B. Each crown 113A is a curved segment or bend extending between opposing struts 113B. The anchoring member 102B is tubular, with the plurality of side openings 114 being defined by edges of the plurality of crowns 113A and the plurality of struts 113B. In an embodiment, the plurality of side openings 114 may be substantially diamond-shaped. The anchoring member 102B includes a plurality of nodes 113C. A node 113C is defined as a region where two crowns of the plurality of crowns 113A within the anchoring member 102B meet or connect. When attached to the valve support 102A via the plurality of connecting components 104, the anchoring member 102B forms an outer frame portion of the frame 102 and the valve support 102A forms an inner frame portion of the frame 102 with the anchoring member 102B circumferentially surrounding the valve support 102 disposed therein.

Each of the valve support 102A and the anchoring member 102B include a skirt or graft material 103A, 103B, respectively, secured thereto. More particularly, the graft material 103A is coupled to an inner surface of the valve support 102A to line a portion thereof. Alternatively, the graft material 103A may be coupled to an outer surface of the valve support 102A to enclose a portion thereof as would be known to one of ordinary skill in the art of prosthetic valve construction. The graft material 103B is coupled to an inner surface of the anchoring member 102B to line a portion thereof. The outer engagement surface of the anchoring member 102 is not covered any sealing or graft material so that the outer engagement surface directly contacts the tissue of the native annulus. The graft material 103A, 103B may be a natural or biological material such as pericardium or another membranous tissue such as intestinal submucosa. Alternatively, the graft material 103A, 103B may be a low-porosity woven fabric, such as polyester, Dacron fabric, or PTFE, which creates a one-way fluid passage when attached to the stent.

The prosthetic heart valve 100 further includes an extension member or brim 115 that extends outwardly from an inflow end of the anchoring member 102B. The brim 115 is formed by a brim support 116 and a flexible web, which in this embodiment is a portion of graft material 103B that extends past or beyond the inflow end of the anchor 102B. More particularly, the graft material 103B (which is coupled to an inner surface of the anchoring member 102B as described above) extends past or beyond the inflow end of the anchoring member 102B, and includes an integral folded pocket or hem beyond the inflow end of the anchoring member 102B. The brim support 116 is disposed within this folded pocket of the graft material 103B. In the depicted embodiment, the brim support 116 includes overlapping, 180 degree out of phase sinusoidal wire forms. However, the brim support 116 may have other configurations. The brim 115 may act as an atrial retainer, if present, and to serve such a function the brim 115 may be configured to engage tissue above a native annulus, such as a supra-annular surface or some other tissue in the right atrium, to thereby inhibit downstream migration of a prosthetic heart valve 100. Accordingly, the brim 115 is of a larger diameter than the frame 102 and extends radially outward from the anchoring member 102B. The portion of graft material 103B connecting the brim 115 to the anchoring member 102B is referred to herein as a brim hinge 117. The brim hinge 117 is configured to permit the brim 115 to hinge and/or flex with respect to the remainder of the prosthetic heart valve 100.

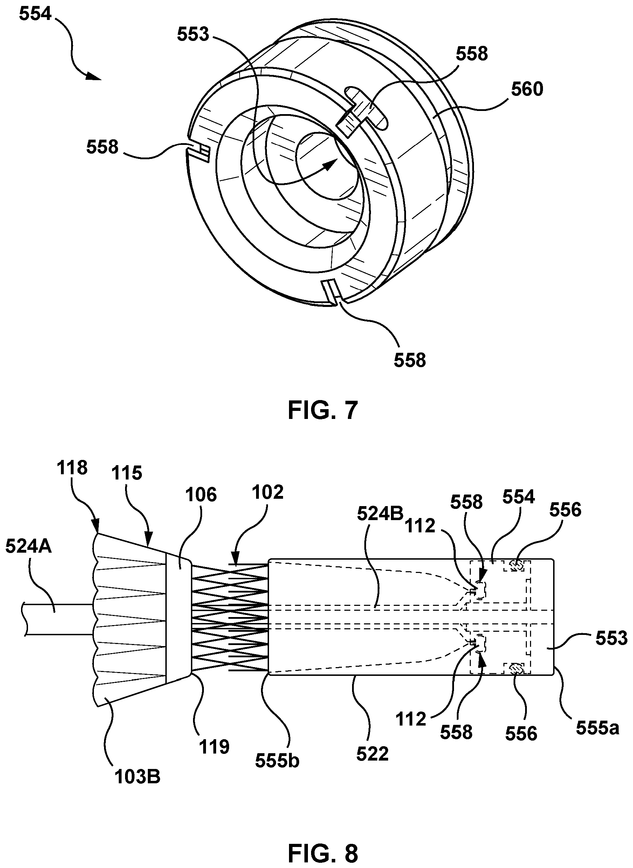

In the depicted embodiment, the brim 115 includes an extension or continuation of the graft material 103B as described above. There is no metal-to-metal connection between the anchoring member 102B and the brim support 116. Thus, the brim 115 is a floppy structure that can readily flex with respect to the anchoring member 102B. However, it is not required that the brim 115 includes an extension or continuation of the graft material 103B. In another embodiment, the brim 115 is a separate component including a flexible web (e.g., graft material or fabric) and the brim support 116 attached thereto, and the brim is attached to the graft material 103B and/or an inflow end of the anchoring member 102B. The brim 115 includes a first end or edge 118 and a second end or edge 119. In operation, the brim 115 guides the prosthetic heart valve 100 during implantation such that the device is located at a desired elevation and centered relative to the native annulus. In some embodiments, one or more components of the brim 115 can be made of or include a radiopaque or echogenic material. For example, as best shown in FIGS. 8, 11, and 12, the brim 115 includes an echogenic band 106 attached thereto, proximate to the second end 119 thereof. The echogenic band 106 enables TEE (trans-esophageal echocardiography) tracking of the prosthetic heart valve 100, and particularly tracking of the movement of the brim 115, *in situ.*

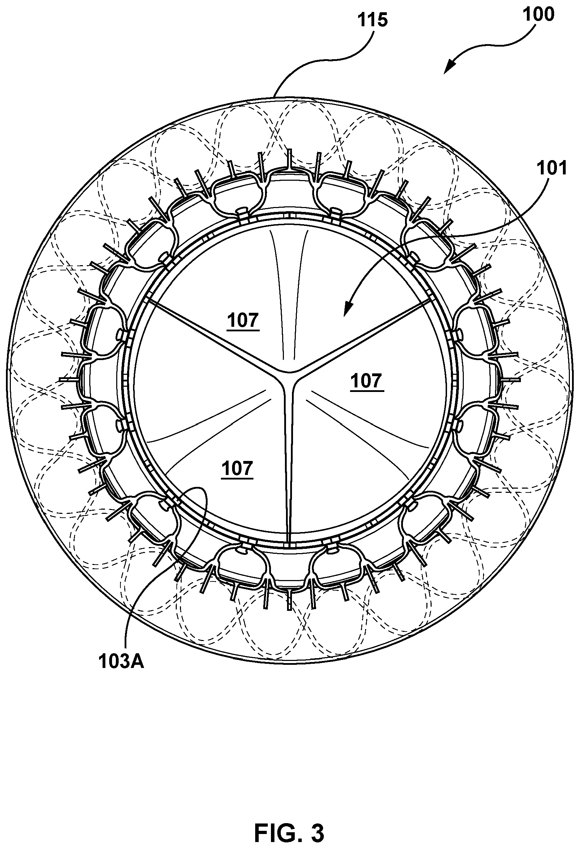

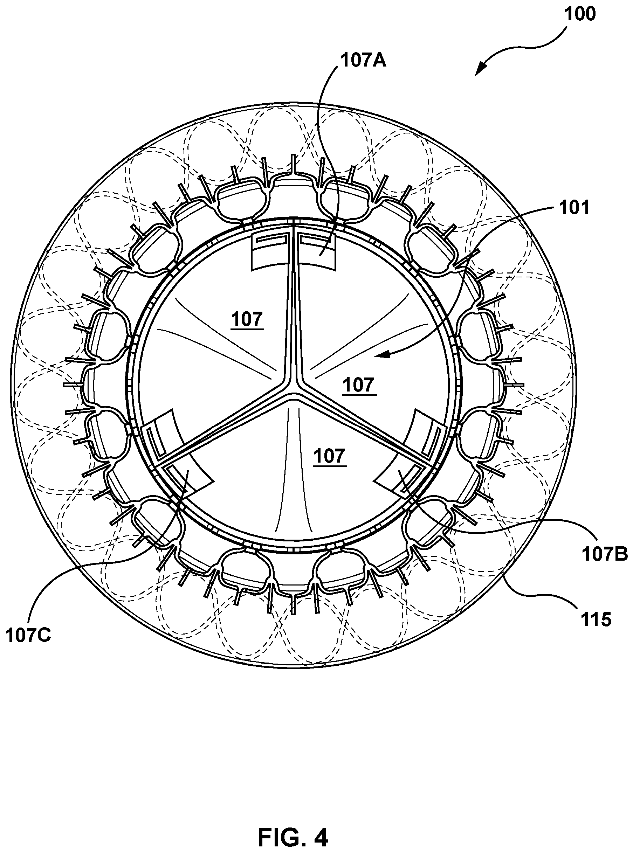

The valve component 101 of the prosthetic heart valve 100 is capable of regulating flow therethrough via valve leaflets 107 that may form a replacement valve. FIGS. 1-4 illustrate an exemplary valve component having three leaflets, although a single leaflet or bicuspid leaflet configuration may alternatively be used in embodiments hereof. When deployed *in situ,* the valve component 101 in a closed state is configured to block blood flow in one direction to regulate blood flow through the central lumen of the valve support 102A. FIG. 2 depicts a perspective view of the valve support 102A with a valve component 101 secured therein, the valve support 102A being shown in FIG. 2 removed from the remainder of the prosthetic heart valve 100 shown in FIG. 1 for ease of illustration. FIG. 3 depicts an atrial or inflow end view of the prosthetic heart valve 100 shown in FIG. 1, and FIG. 4 depicts a ventricular or outflow end view of the prosthetic heart valve 100 shown in FIG. 1. The valve component 101 includes valve leaflets 107, e.g., three valve leaflets 107, that are disposed to coapt within an upstream portion of the valve support 102A with leaflet commissures 107A, 107B, 107C of the valve leaflets 107 being secured within a downstream portion of the valve support 102A, such that the valve leaflets 107 open during diastole. Leaflets 107 are attached along their bases to the valve support 102A, for example, using sutures or a suitable biocompatible adhesive. Adjoining pairs of leaflets 107 are attached to one another at their lateral ends to form leaflet commissures 107A, 107B, 107C. The orientation of the leaflets 107 within the valve support 102A depends upon on which end of the prosthetic heart valve 100 is the inflow end and which end of the prosthetic heart valve 100 is the outflow end, thereby ensuring one-way flow of blood through the prosthetic heart valve 100.

The valve leaflets 107 are attached to the graft material 103A in order to form the valve component 101. The valve leaflets 107 may be formed of various flexible materials including, but not limited to natural pericardial material such as tissue from bovine, equine or porcine origins, or synthetic materials such as polytetrafluoroethylene (PTFE), DACRON^{®} polyester, pyrolytic carbon, or other biocompatible materials. With certain prosthetic leaflet materials, it may be desirable to coat one or both sides of the replacement valve leaflet with a material that will prevent or minimize overgrowth. It is further desirable that the prosthetic leaflet material is durable and not subject to stretching, deforming, or fatigue.

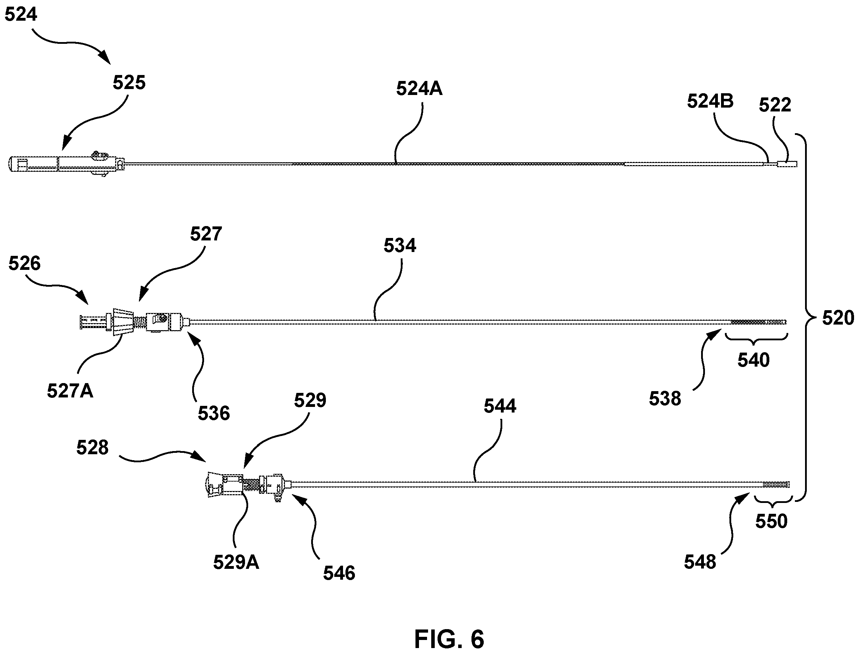

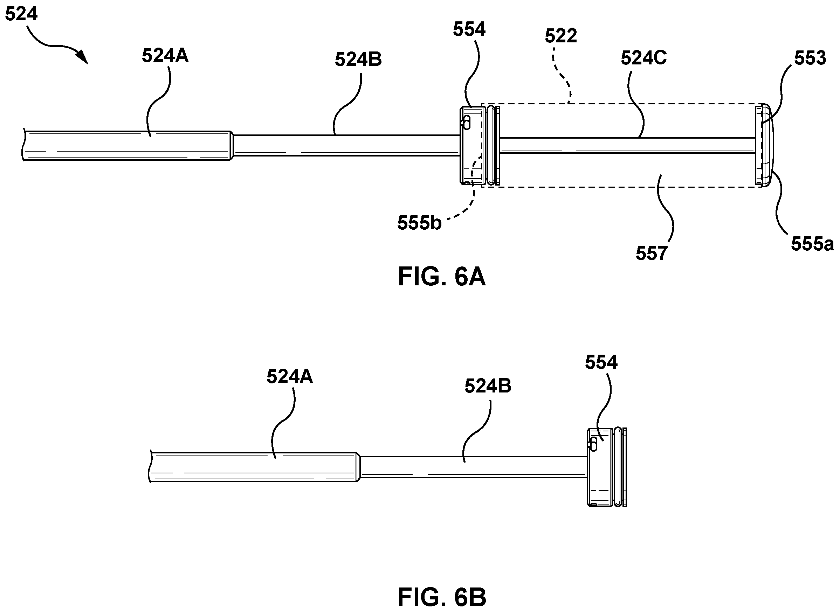

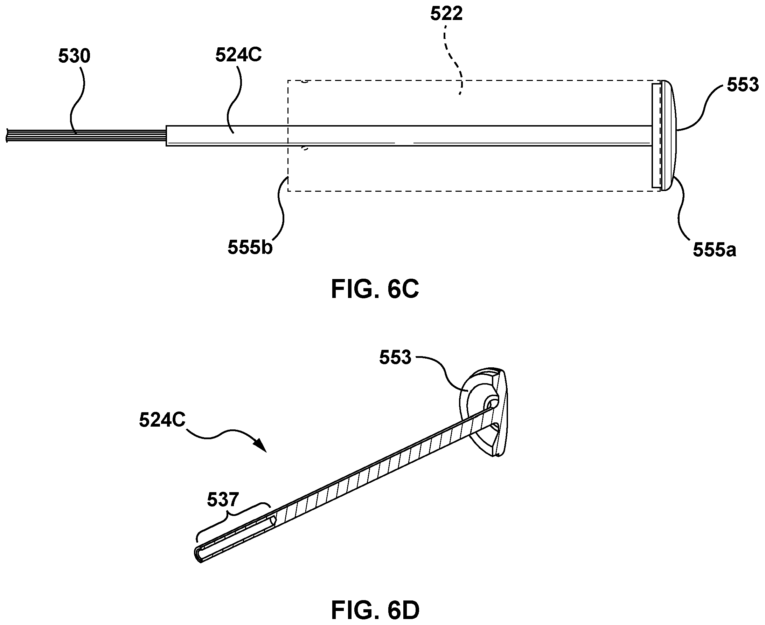

A delivery system 520 which may be used for transcatheter delivery and deployment of an implant, such as the non-limiting example of the prosthetic heart valve 100 of FIGS. 1-4, is shown in FIGS. 5-6D. In general terms, the delivery system 520 is arranged and configured for percutaneously delivering an implant (e.g., prosthetic heart valve 100) in a delivery configuration to a patient's native defective heart valve or other portion of a patient's anatomy via transcatheter delivery. FIG. 5 illustrates a side view of the delivery system 520, and FIG. 5A is a cross-sectional view taken along line A-A of FIG. 5. FIG. 6 is an exploded view of the delivery system 520. The delivery system 520 includes a capsule 522 for housing at least a portion of the prosthetic heart valve 100, an innermost shaft assembly 524 contained within and coupled to the capsule 522, an inner steerable catheter 526 disposed over the innermost shaft assembly 524, and an outer steerable catheter 528 disposed over the inner steerable catheter 526. The inner steerable catheter 526 includes a handle 527 at a proximal portion thereof for manipulation *in situ,* and the outer steerable catheter 528 includes a handle 529 at a proximal portion thereof for manipulation *in situ.* During delivery, the prosthetic heart valve 100 contained within the capsule 522 is steered by the inner steerable catheter 526 and the outer steerable catheter 528 into alignment within the tricuspid valve for which the prosthetic heart valve 100 serves as a replacement. The inner steerable catheter 526 may be controlled or steered independently from the outer steerable catheter 528, and provides the delivery system 520 with omnidirectional steering capabilities to direct the capsule 522.

Components of the delivery system 520 will now be described in more detail. At a proximal end thereof, as best shown in the exploded view of FIG. 6, the innermost shaft assembly 524 is fixedly secured to a manifold 525. FIG. 6A is an enlarged view of a distal portion of the innermost shaft assembly 524. The innermost shaft assembly 524 includes a flexible shaft 524A, a piston mount 524B, a piston 554, a tension cable 530, a distal shaft 524C, a capsule 522, and a capsule cap 553. The innermost shaft assembly 524 may be considered to include a first subassembly, shown in FIG. 6B and which includes the flexible shaft 524A, the piston mount 524B and the piston 524, and a second subassembly, shown in FIG. 6C and which includes the tension cable 530, the distal shaft 524C, the capsule 522, and the capsule cap 533. The first and second subassemblies are coupled together in that the distal shaft 524C of the second subassembly slides or telescopes within the piston mount 524B of the first subassembly. In addition, the first and second subassemblies are coupled together via the manifold 525.

With respect to the first subassembly shown in FIG. 6B, the flexible shaft 524A is a flexible elongated tubular body that may include, for example, a flexible metal tetris or spring disposed within a polymer jacket. A distal end of the flexible shaft 524A is attached and fixed relative to a proximal end of the piston mount 524B, which is a rigid, tubular body that distally extends from the flexible shaft 524A. The piston 554 is attached and fixed relative to the piston mount 524B. As will be explained in more detail herein with respect to FIG. 7, the piston 554 is disposed over and mounted to a distal end of the piston mount 524B.

With respect to the second subassembly shown in FIG. 6C, the capsule 522 is a tubular component having a closed or distal end 555a and an open or proximal end 555b. The capsule 522 may be rigid and made of metal. As will be described in more detail herein with respect to FIGS. 7-10, the capsule 522 capsule 522 is configured to house at least a portion of the prosthetic heart valve 100 during delivery. The distal end 555a of the capsule 522 is closed via the capsule cap 553. The capsule cap 553 may be integrally formed with the capsule 522 or may be a separate component attached thereto to form the closed distal end 555a. The distal shaft 524C is further attached to the capsule cap 553. FIG. 6D is a sectional view of the distal shaft 524C and the capsule cap 553. As shown in FIG. 6D, the distal shaft 524C may be integrally formed with the capsule cap 553, or in another embodiment, the distal end of the distal shaft 524C may be welded or otherwise attached to the capsule cap 553.

With additional reference to the cross-sectional view of FIG. 5A and the sectional view of FIG. 5B, the tension cable 530 extends from the manifold 525 to the distal shaft 524C through the lumens of the flexible shaft 524A and the piston mount 524B. The lumens of the flexible shaft 524A and the piston mount 524B are in fluid communication with each other, and are designated with the reference number 531 on FIG. 5A. A distal end of the tension cable 530 is secured or mounted within a proximal portion 537 of the distal shaft 524C. In an embodiment, the tension cable 530 is configured to be selectively tensioned (proximally or distally) by hydraulic force to enable translation of the capsule 522 either proximally or distally with respect to the piston mount 524B and the piston 554 attached thereto. Depending on which hydraulic system is engaged (deployment or recapture), the tension cable 530 will translate under tension through the innermost shaft assembly 524, with the capsule 522 moving distally during deployment or proximally during recapture.

More particularly, as best shown in the sectional view of FIG. 5B, the manifold 525 includes a recapture piston 590 and a recapture chamber 591. The recapture chamber 591 is disposed within the manifold 525 and is configured to house the recapture piston 590 therein. The manifold also includes a deployment valve 594 that is configured to be coupled to a first external source or pump 595 of hydraulic fluid and a recapture valve 592 that is configured to be coupled to a second external source or pump 593 of hydraulic fluid. Hydraulic fluid is delivered from the pump 595 through the deployment valve 594 in order to drive the capsule 522 in a distal direction, thereby deploying the prosthetic heart valve 100, as will be described in more detail herein with respect to FIGS. 7-10. Hydraulic fluid is delivered to from the pump 593 through the recapture valve 592 of the delivery device 520 in order to drive the capsule 522 in a proximal direction, thereby recapturing the prosthetic heart valve 100 if desired or necessary. More particularly, the recapture chamber 591 is fluidly coupled to the recapture valve 592 and is configured to fill with hydraulic fluid. As the hydraulic fluid flows into and/or exits from the recapture chamber 591, the recapture piston 590 is configured to move axially within the recapture chamber 591. Although the system is shown with two pumps 593, 595, in another embodiment hereof, a single external source or pump may be utilized as both first pump 595 and second pump 593. For example, a single inflation device may be connected to a flow reverser that enables the operator to toggle between either a deployment or recapture position. The flow reverser diverts the fluid flow either to the deployment valve 594 or the recapture valve 592, depending on the desired mode of operation.

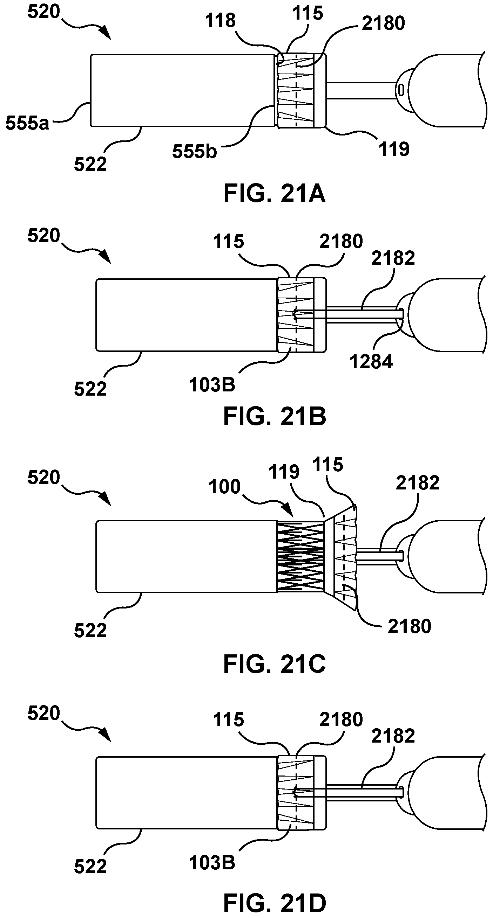

The recapture piston 590 is operably coupled to the capsule 522 via the tension cable 530, meaning that movement of either will place an axial force onto the opposing component. More particularly, a proximal end of the tension cable 530 is attached to the recapture piston 590 and a distal end of the tension cable 530 is attached the distal shaft 524C, which is, subsequently coupled to the capsule cap 553 as described above. The tension cable 530 remains taut or under tension between the recapture piston 590 and the distal shaft 524C, resulting in a force that encourages the recapture piston 590 and distal shaft 524C to move together. Prior to deployment of the prosthetic heart valve 100, the recapture piston 590 is disposed at a proximal end of the recapture chamber 590 and the recapture chamber 591 is filled with hydraulic fluid. As the capsule 522 is driven distally as described in more detail with reference to FIGS. 7-10, the recapture piston 590 is also pulled distally since the proximal end of the tension cable is attached thereto and hydraulic fluid within the recapture chamber 591 is pushed out of the recapture chamber 591 as the recapture piston 590 is pulled distally during deployment of the prosthetic heart valve 100. However, it may be desired to recapture the prosthetic heart valve 100 after deployment thereof as described in more detail with respect to FIGS. 21A-21D. During recapture, hydraulic fluid is delivered from the pump 593 through the recapture valve 592 of the delivery device 520 in order to drive the capsule 522 in a proximal direction, thereby recapturing the prosthetic heart valve 100.

The distal shaft 524C is received within the lumen of the piston mount 524B and may move or slide relative thereto in an axial or longitudinal direction. Stated another way, the distal shaft 524C telescopes within the piston mount 524B. The capsule 522 is concentrically disposed over the distal shaft 524C, and an annular chamber 557 (shown in FIG. 6A) is defined between an inner surface of the capsule 522, an outer surface of the distal shaft 524C, the piston 554 and the capsule cap 553. As will be described in more detail with respect to FIGS. 7-10, the annular chamber 557 is part of a hydraulic deployment system that is configured to cause proximal and distal translation of the capsule 522 with respect to the prosthetic heart valve 100 for deployment thereof. The annular chamber 557 is a sealed cavity formed by the piston 554, the seal 556, the capsule 552, and the capsule cap 553 into which fluid can be introduced to increase fluid pressure therein and thereby move the capsule 522 away from the piston 554, which remains stationary during fluid delivery as described below.

The inner steerable catheter 526 is disposed over the innermost shaft assembly 524 such that an annular lumen 532 (shown on FIG. 5A) is defined between an outer surface of the innermost shaft assembly 524 and an inner surface of the inner steerable catheter 526 along an entire length of the inner steerable catheter 526. The innermost shaft assembly 524 is slidingly disposed within the inner steerable catheter 526 such that relative axial movement is permitted therebetween as will be described in more detail below. As used herein, "slidably" generally denotes back and forth movement in a longitudinal direction along or generally parallel to a central longitudinal axis L_{A} of the delivery system 520. The inner steerable catheter 526 includes a flexible, steerable tubular component or shaft 534, the handle 527 fixedly secured to a proximal end 536 of the shaft 534, an inner distal flex component 540 extending distally from a distal end 538 of the shaft 534, and a first pullwire 542. The shaft 534 can assume various forms conventionally employed, and in some embodiments can be a braided catheter surrounded by a polymer outer layer or jacket. The inner distal flex component 540 is secured to and extends distally from the shaft 534, and can be configured to exhibit flexibility and/or hoop strength characteristics differing from that of the shaft 534. In an embodiment, the inner distal flex component 540 is a metal tube with a laser cut pattern that facilitates flexing or bending of the inner distal flex component 540. A cap 541 is attached to a distal end of the inner distal flex component 540. The cap 541 is an annular component that permits the innermost shaft assembly 524 to slide therethrough. The handle 527 includes an actuator 527A that is accessible to the user and may be manipulated to control flexing or bending of the inner distal flex component 540 of the shaft 534. More particularly, as will be explained in more detail herein, the first pullwire 542 is attached to and extends between the handle 527 and the cap 541 attached to the inner distal flex component 540. The first pullwire 542 is selectively tensioned by the user to bend the inner distal flex component 540. The inner steerable catheter 526 is configured to transition between a non-flexed configuration when the first pullwire 542 is not tensioned and a flexed configuration in which the first pullwire 542 is tensioned.

The handle 527 includes the actuator 527A for tensioning the first pullwire 542. The handle 527 can have any shape or size appropriate for convenient handling by a user. The actuator 527A is coupled to the proximal end of the first pullwire 542, and is generally constructed to provide selective proximal retraction and distal advancement of the first pullwire 542. Stated another way, the actuator 527A is coupled to the proximal end of the first pullwire 542 and is constructed to selectively push or pull the first pullwire 542. The actuator 527A may assume any construction that is capable of providing the desired pullwire actuation functionality. In an embodiment, the actuator 527A is configured as a rotatable knob that is rotated in a first direction (i.e., clockwise) to proximally retract the first pullwire 542 and apply tension thereto, and is rotated in a second, opposing direction (i.e., counter-clockwise) to distally advance the first pullwire 542 and remove or release tension therefrom, such as the rotatable knob described in U.S. Patent No. 10,188,833 to Bolduc et al., filed December 8, 2015, or the rotatable knob described in U.S. Patent No. 6,607,496 to Poor et al., filed on September 12, 2000, each of which is assigned to the same assignee as the present disclosure. In another embodiment, the actuator 527A may be configured as a button such as those described in U.S. Patent No. 10,278,852 to Griffin, filed on March 10, 2016, which is assigned to the same assignee as the present disclosure**.**

The outer steerable catheter 528 is slidably disposed over the inner steerable catheter 526 such that an annular lumen 543 (shown on FIG. 5A) is defined between an outer surface of the inner steerable catheter 526 and an inner surface of the outer steerable catheter 528 along an entire length of the outer steerable catheter 528. The outer steerable catheter 528 includes a flexible, steerable tubular component or shaft 544, the handle 529 fixedly secured relative to a proximal end 546 of the shaft 544, an outer distal flex component 550 extending distally from a distal end 548 of the shaft 544, and a second pullwire 552. The shaft 544 can assume various forms conventionally employed, and in some embodiments can be a braided catheter surrounded by a polymer outer layer or jacket. The outer distal flex component 550 is secured to and extends distally from the shaft 544, and can be configured to exhibit flexibility and/or hoop strength characteristics differing from that of the shaft 544. In an embodiment, the outer distal flex component 550 is formed from a metal tube with a laser cut pattern that facilitates flexing or bending of the outer distal flex component 550. A cap 551 is attached to a distal end of the outer distal flex component 550. The cap 551 is an annular component that permits the inner steerable catheter 526 to slide therethrough. The handle 529 includes an actuator 529A that is accessible to the user and may be manipulated to control steering of the outer distal flex component 550 of the shaft 544. More particularly, as will be explained in more detail herein, the second pullwire 552 is attached to and extends between the handle 529 and the cap 551 of the outer distal flex component 550. The second pullwire 552 is selectively tensioned by the user to bend the outer distal flex component 550. The outer steerable catheter 528 is configured to transition between a non-flexed configuration when the second pullwire 552 is not tensioned and a flexed configuration in which the second pullwire 552 is tensioned.

The handle 529 includes the actuator 529A for tensioning the second pullwire 552. The handle 529 can have any shape or size appropriate for convenient handling by a user. The actuator 529A is coupled to the proximal end of the second pullwire 552, and is generally constructed to provide selective proximal retraction and distal advancement of the second pullwire 552. Stated another way, the actuator 529A is coupled to the proximal end of the second pullwire 552 and is constructed to selectively push or pull the second pullwire 552. The actuator 529A may assume any construction that is capable of providing the desired pullwire actuation functionality. In an embodiment, the actuator 529A is configured as a rotatable knob that is rotated in a first direction (i.e., clockwise) to proximally retract the second pullwire 552 and apply tension thereto, and is rotated in a second, opposing direction (i.e., counter-clockwise) to distally advance the second pullwire 552 and remove or release tension therefrom, such as the rotatable knob described in U.S. Patent No. 10,188,833 to Bolduc et al., filed December 8, 2015, or the rotatable knob described in U.S. Patent No. 6,607,496 to Poor et al., filed on September 12, 2000, each of which is assigned to the same assignee as the present disclosure**.** In another embodiment, the actuator 529A may be configured as a button such as those described in U.S. Patent No. 10,278,852 to Griffin, filed on March 10, 2016, which is assigned to the same assignee as the present disclosure**.**

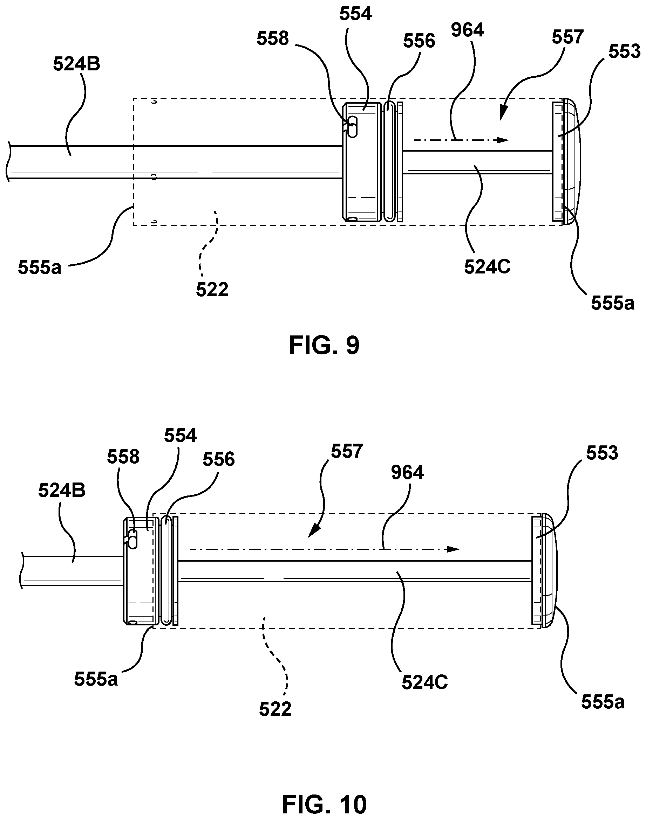

Turning now to FIGS. 7-10, the operation of the capsule 522 for radially compressing and deploying the capsule 522 will be described in more detail. FIG. 7 is a perspective view of the piston 554. FIGS. 8, 9, and 10 illustrate various positions of the capsule 552 relative to the piston 554 during deployment of the prosthetic heart valve 100. The prosthetic heart valve 100 is depicted in FIG. 8, but is not shown in FIGS. 9 and 10 for sake of clarity, as these figures are primarily provided to illustrate the relative movement of the capsule 552 relative to the piston 554.

The piston 554 is shown removed from the delivery system 520 in FIG. 7. The piston 554 is an annular component that defines an opening or central bore 533 such that the piston 554 is configured to be disposed over and attached to a distal end of the piston mount 524B. The piston 554 includes a plurality of slots or recesses 558 configured to receive the attachment bars 112 of the prosthetic heart valve 100, as shown in FIG. 8. The piston 554 also includes an annular groove 560 on an outer surface thereof. A seal 556 (shown in FIGS. 8-10) is disposed within the annular groove 560. The seal 556 is thus coupled to the piston 554 and functions to provide a fluid seal between the piston 554 and an inner surface of the capsule 522. The seal 556 may be, for example, an O-ring. When fluid is present in the annular chamber 557, the seal 556 prevents fluid from leaking out between the outer surface of the piston 554 and the inner surface of the capsule 522.

Referring now to FIG. 8, a side view of the distal portion of the delivery system 520 is shown. The distal portion includes the capsule 522 and the piston 554. In FIG. 8, the capsule 522 is in a first position relative to the piston 554 in which the piston 554 abuts against or is disposed directly adjacent to the capsule cap 553. In this first position, the capsule 522 is positioned so as to compressively retain at least a portion of the prosthetic heart valve 100. The prosthetic heart valve 100 is coupled to the piston 554 via the attachment bars 112 being disposed within the plurality of slots 558. Notably, the capsule 522 has a length that is shorter than a length of the prosthetic heart valve 100. In this configuration, which may be considered an initial loading configuration, the brim 115 is not yet everted (extends away from the frame 102) and the frame 102 of the prosthetic heart valve 100 is radially compressed by the capsule 522. In FIG. 8, the distal shaft 524C is concealed from view since the piston mount 524B extends thereover.

With additional reference to FIGS. 9 and 10, the capsule 522 is configured to be distally advanced relative to the piston 554 in order to incrementally release and deploy the frame 102 of the prosthetic heart valve 100 from the capsule 522. Via the manifold 525, fluid is injected through the innermost shaft assembly 524 in order to drive the capsule 522 distally. The prosthetic heart valve 100 may remain in a stationary longitudinal position relative to the native valve while the capsule 522 is driven distally, thereby increasing the precision of deployment. Hydraulic valve delivery systems consistent with embodiments hereof include, for example, those described in U.S. Patent No. 9,034,032 to McLean et al.*,* International Patent Application No. PCT/US5114/029549 to McLean et al.*,* and U.S. Patent No. 10,561,497 to Duffy et al.

More particularly, the manifold 525 may be connected to an external fluid source (not shown). The external fluid source is fluidly connected to the annular chamber 557 within the capsule 522 via the lumens of the flexible shaft 524A and the piston mount 524B (which are in fluid communication with each other). Fluid enters the annular chamber 557 via the outlet of the piston mount 524B, around the distal shaft 524C through the annular space or lumen 531 (see FIG. 5a) defined between the outer surface of the distal shaft 524C and the inner surface of the piston mount 524B. As shown by the directional arrow 964 in FIG. 9, as the annular chamber 557 fills with fluid, the capsule 522 is distally advanced with respect to the piston 554. FIG. 9 illustrates the capsule 522 at a second position relative to the piston 554, in which the piston 554 is disposed within the capsule 522 at approximately a midportion thereof. The annular chamber 557 between the piston 554 and the capsule cap 553 is filled with fluid from the external fluid source. The piston 554, which is attached and fixed to the piston mount 524B and the flexible shaft 524A, remains stationary as the capsule 522 and distal shaft 524C move in an axial direction. The piston 554 (and piston mount 524B and flexible shaft 524A) may be held in place by holding the manifold 525 stationary during fluid delivery.

The fluid continues to fill the annular chamber 557 until the capsule 522 reaches a third position relative to the piston 554 depicted in FIG. 10, in which the piston 554 is partially disposed within the capsule 522 and is directly adjacent to the proximal end 555b of the capsule 522. The annular chamber 557 between the piston 554 and the capsule cap 553 is filled with fluid from the external fluid source. At this third position, the plurality of slots 558 of the piston 554 are no longer covered by the capsule 552. With the plurality of slots 558 exposed, the attachment bars 112 of the prosthetic heart valve 100 are permitted to decouple from the piston 554. Accordingly, via movement of the capsule 552, the prosthetic heart valve 100 is unsheathed from the capsule 522. When the capsule 522 no longer covers or extends over the attachment bars 112, the attachment bars 112 are free or permitted to pop out of the slots 558 of the piston 554 to decouple the prosthetic heart valve 100 from the piston 554. Thus, once the prosthetic heart valve 100 is no longer covered by the capsule 522, the prosthetic heart valve 100 is permitted to radially self-expand towards the expanded configuration of FIG. 1.

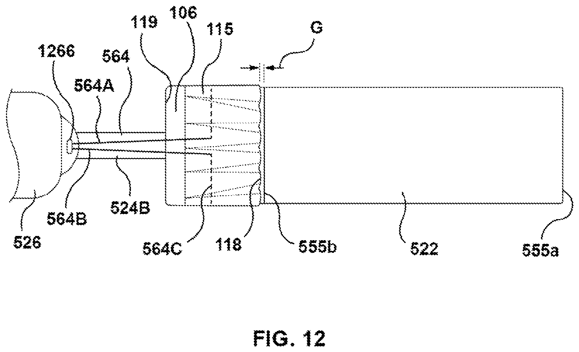

Turning now to FIGS. 11 and 12, a delivery configuration of the prosthetic heart valve 100 will be described with respect to the delivery system 520. FIG. 11 depicts a similar view to FIG. 8. In FIG. 11, the capsule 522 is positioned so as to compressively retain at least a portion of the prosthetic heart valve 100. In this embodiment, the capsule 522 has a uniform outer diameter and a uniform thickness along its entire length. Notably, the capsule 522 has a length that is shorter than a length of the prosthetic heart valve 100. In this configuration, which may be considered an initial loading configuration, the brim 115 is not yet everted (extends away from the frame 102) and the frame 102 of the prosthetic heart valve 100 is radially compressed by the capsule 522. In one example, the capsule 522 has a length L1 of about 30 mm (+/- 2 mm) and the frame 102 has a length of about 38 mm (+/-2 mm) when radially compressed in the delivery configuration. The brim 115 has a length of about 9 mm (+/- 2 mm), and in the initial loading configuration is reverted and extends away from the frame 102. The length L1 of the capsule 522 is between 70% - 80% of the length of the frame 102 when the prosthetic valve 100 is radially compressed. The proximal end 555b of the capsule 522 is spaced apart from the brim 115 by a length L2 of about 9 mm (+ 0 mm/- 2 mm). Stated another way, the length L2 of the uncovered portion of the frame 102 is between 20% - 30% of the length of the frame 102 when the prosthetic valve 100 is radially compressed. In this initial loading configuration, between the proximal end 555b of the capsule 522 and the brim 115, the frame 102 of the heart valve prosthesis 100 is exposed or not covered by the capsule 522. The portion of the frame 102 that is exposed or not covered by the capsule 522 in this initial loading configuration is referred to herein as uncovered portion 1162.

As shown in FIG. 12, the brim 115 is configured to distally fold or evert in order to cover the uncovered portion 1162, thereby resulting in a final loading or a delivery configuration of the system. Thus, during delivery within a vasculature, a portion of the prosthetic heart valve 100 is used to sheathe a portion of the frame 102. More particularly, the brim 115 of the prosthetic heart valve 100 is everted so that both the brim 115 and the capsule 522 collectively sheathe the anchoring member 102B to a degree such that the anchoring member 102B is not exposed. Utilizing the brim 115 to sheathe a portion of the anchoring member 102B during delivery allows for the length of the capsule 522 to be reduced, while still sheathing the anchoring member 102B to provide atraumatic delivery. The relatively shortened capsule 522 is particularly beneficial for tricuspid valve replacement procedures. Access to a tricuspid valve can be challenging in that existing implanted devices may be in the anatomy, reducing the space available for the delivery system. In addition, visualization of the delivery system and the prosthetic heart valve 100 may be challenging as metallic capsules can cause artifacts due to density. Further, chordae and papillary muscles serve as obstacles for delivery and the right ventricle is generally shorter than the left ventricle. All of these considerations result in a general desire for a system capable of delivering an implant to a tricuspid valve while reducing a length the delivery system extends into the ventricle and past the valve annulus. When deploying the prosthetic heart valve 100, it is desirable to avoid contact between the capsule and the inside of the right ventricle. The anatomy and dimensions of a patient's right ventricle vary, and thus it is desirable to minimize the length that the delivery system extends into the ventricle and past the valve annulus in order to result in a higher number of patients that may be treated. In one non-limiting example, the delivery system 520 will require approximately 63 mm of access in the right ventricle to achieve full deployment of the prosthetic heart valve 100 as compared to a current requirement of about 70 mm for a capsule that fully sheathes the prosthetic heart valve 100. This approximately 7 mm reduction in required deployment depth will result in a higher number of patients having access to the present technology. Stated another way, the approximately 7 mm reduction allows the delivery system 520 to be configured to treater relatively shorter right ventricles and therefore a higher number of patients. Additionally, the present inventors believe that a shorter capsule facilitates easier steering of the capsule 522 and enable the delivery system 520 to be delivered to an annulus with greater ease and less potential for trauma to the anatomy. It will be understood that these benefits may also apply to other heart valve replacement procedures, including mitral heart valve replacement procedures.

It will be understood by one of ordinary skill in the art that the dimensions of the lengths L1 and L2 described above with respect to FIG. 11 are exemplary only. The length L1 of the capsule 522 may be greater or less than the length shown in FIG. 11, and the length L2 of the exposed portion of the frame 102 may be greater or less than the length shown in FIG. 11. In another embodiment, the length L1 of the capsule 522 is between 50% and 70% of the length of the frame 102 when the prosthetic valve 100 is radially compressed and the length L2 of the uncovered portion of the frame 102 is between 30% and 50% of the length of the frame 102 when the prosthetic valve 100 is radially compressed. For example, with reference to FIG. 11A, an embodiment of a capsule 1122B is shown in which the length L1 of the capsule 1122B is about 60% of the length of the frame 102 when the prosthetic valve 100 is radially compressed and the length L2 of the uncovered portion of the frame 102 is about 40% of the length of the frame when the prosthetic valve 100 is radially compressed. It will be apparent to one of ordinary skill in the art that a variety of factors including the intended application and target site, intended vasculature route for deployment, configuration of the prosthetic heart valve, configuration and dimensions of the brim, and the like may determine the relative dimensions of the lengths L1 and L2.

When everted and in the delivery configuration, the brim 115 covers at least some of the fixation elements 105 of the anchoring member 102B. For example, the brim 115 may cover a first row of fixation elements 105 that are disposed closest to the first or inflow end 108 of the prosthetic heart valve 100. In another example, the brim 115 may cover the first two rows of fixation elements 105 that are disposed closest to the first or inflow end 108 of the prosthetic heart valve 100. As stated above, when the prosthetic heart valve 100 is in the expanded configuration of FIG. 1, the fixation elements 105 project radially outward and are inclined toward an upstream direction. However, when the capsule 522 is positioned so as to compressively retain at least a portion of the prosthetic heart valve 100 and the brim 115 is everted and disposed over the fixation elements 105, the fixation elements 105 may flatten towards a more longitudinal orientation such that the covered fixation elements 105 extend generally parallel to the longitudinal axis of the capsule 522. When the brim 115 reverts as described in more detail herein, the fixation elements 105 resume their expanded configuration in which they project radially outward and are inclined toward an upstream direction. It will be apparent to one of ordinary skill in the art that the number of rows of fixation elements 105 may vary from that shown in FIG. 1. In another embodiment (not shown), the prosthetic heart valve 100 includes only a single row of fixation elements 105 and the single row may be disposed on nodes 113C that are closest to the first or inflow end 108 of the prosthetic heart valve 100. In another embodiment (now shown), the prosthetic heart valve 100 includes exactly two rows of fixation elements 105 and the two rows may be disposed on nodes 113C that are closest to the first or inflow end 108 of the prosthetic heart valve 100. Further, in yet another embodiment, fixation elements 105 may be eliminated and the prosthetic heart valve 100 may be anchored solely via the self-expanding material of the frame 102. In an embodiment, the frame 102 of the prosthetic heart valve 100 may be oversized relative to the diameter of the native valve annulus to ensure that the outward radial force of the frame 102 in the expanded configuration is sufficient to secure the prosthetic heart valve 100 within the native valve annulus *in situ.*

The delivery system 520 includes a brim lock 564 to aid in holding the brim 115 in the everted position. The brim lock 564 may be a removable suture that wraps around the brim 115 when everted to hold the brim 115 in the everted position. The brim lock 564 may be disposed within the integral folded pocket or hem of the graft material 103B, or may be woven through the graft material 103B of the brim 115. When tension is applied and sustained to the brim lock 564, the brim lock 564 tightens around the brim 115 to apply an inward radial force that contains the brim 115 in the everted position. At the desired point in time, to initiate deployment of the prosthetic heart valve 100, the brim lock 564 can removed to release the brim 115, allowing the brim to revert.

More particularly, the brim lock 564 may be a single, continuous elongated component that runs from the handle 527 of the inner steerable catheter 526 to the brim 115 of the prosthetic heart valve 100, around the brim 115 of the prosthetic heart valve 100, and back from the prosthetic heart valve 100 to the handle 527 of the inner steerable catheter 526 so that both ends of the brim lock 564 are accessible to the user. The brim lock 564 may extend within the inner steerable catheter 526 via a port 1266. The brim lock 564 is releasable to permit the brim 115 of the prosthetic heart valve 100 to return to an expanded or deployed state. More particularly, pulling on one or both ends of the brim lock 564 controls constriction/compression of the brim 115 of the prosthetic heart valve 100 and releasing/removing the brim lock 564 controls expansion/deployment of the brim 115 of the prosthetic heart valve 100. In an embodiment, the brim lock 564 may be formed from a monofilament or plastic suture material, such as polypropylene or polyethylene.

When placed within the delivery system 520, the brim lock 564 integrally includes a first leg 564A, a second leg 564B, and a loop 564C formed therebetween the first and second legs 564A, 564B. The proximal ends of the first and second legs 564A, 564B may extend proximally out of the handle 527 so as to be accessible to the user. The delivery system 520 also includes a dual lumen tube 565 (see FIG. 5A) for housing the brim lock 564. The dual lumen tube 565 extends between the handle 527 of the inner steerable catheter 526 and a distal end of the inner distal flex component 540. The dual lumen tube 565 extends within the annular lumen 532 defined between an outer surface of the innermost shaft assembly 524 and an inner surface of the inner steerable catheter 526. The dual lumen tube 565 thus extends alongside or adjacent to the inner surface of the inner distal flex component 540 and alongside or adjacent to the inner surface of the shaft 534 for the entire length of the inner steerable catheter 526. A proximal end (not shown) of the dual lumen tube 565 is fixedly secured to the handle 527 and does not move relative thereto. The first leg 564A of the brim lock 564 extends through the first lumen of the dual lumen tube 565, and the second leg 564B of the brim lock 564 extends through the second lumen of the dual lumen tube 565. Separate or dedicated lumens for each leg 564A, 564B of the brim lock 564 reduces twisting, entanglement, and friction of the suture legs within the delivery system 520. The loop 564C of the brim lock 564 is disposed distally of the distal end of the dual lumen tube 565, and extends around the brim 115 of the prosthetic heart valve 100. The loop 564C of the brim lock 564 encircles or extends circumferentially around the brim 115 of the prosthetic heart valve 100 and is configured to hold the brim 115 in a reduced diameter state for delivery to the treatment site. The brim lock 564 is removed from the prosthetic heart valve 100 by pulling on one end of the brim lock 564 (either the end associated with the first leg 564A or the end associated with second leg 564B) until the entire brim lock 564 is pulled through and removed from the delivery system 520.

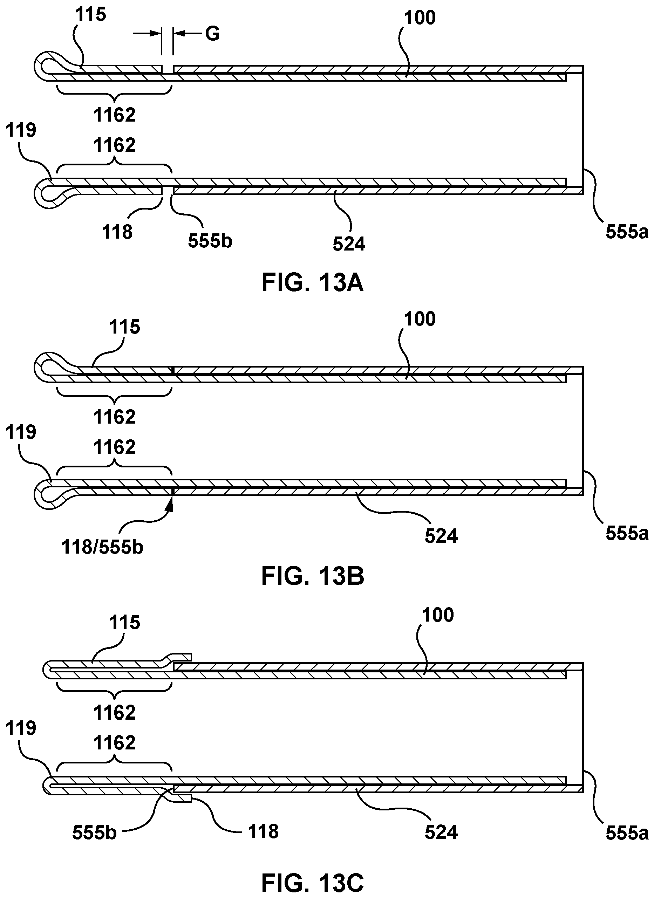

When everted and in the delivery configuration, in the embodiment of FIG. 12, the first end 118 of the brim 115 is directly adjacent to and proximal to the proximal end 555b of the capsule 522 so that the brim 115 and capsule 522 are not overlapping. "Not overlapping" as used herein includes the first end 118 of the brim 115 is slightly spaced apart from the proximal end 555b of the capsule 522 by a gap G, as shown in FIGS. 12 and 13A, as well as the first end 118 of the brim 115 abutting against the proximal end 555b of the capsule 522, as shown in FIG. 13B. In another embodiment, shown in FIG. 13C, the brim 115 overlaps with the capsule 522 over an outer surface of the capsule 522. The amount of overlap may be between 1 mm and 2 mm (+1/-0 mm).

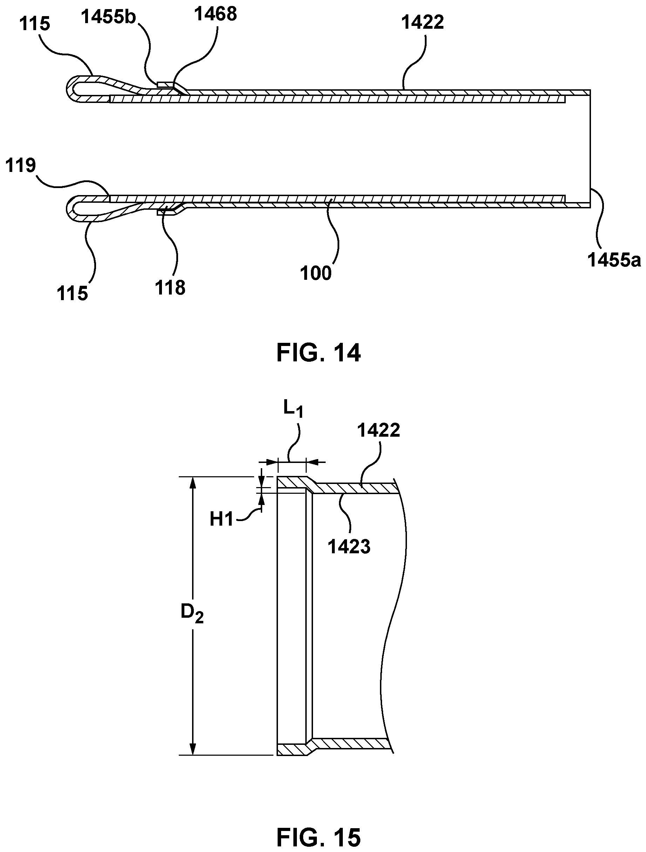

The capsule may include a raised lip for tucking the brim under the proximal end of the capsule, and the raised lip assists in holding the brim 115 in the everted position. The raised lip may be utilized in conjunction with the brim lock 564 to hold the brim 115 in the everted position, or may be utilized as an alternative to the brim lock 564 to hold the brim 115 in the everted position. More particularly, FIGS. 14 and 15 depict a capsule 1422 according to another embodiment hereof that may be utilized within the delivery system 520. Similar to the capsule 522, the capsule 1422 is a tubular component having a closed or distal end 1455a and an open or proximal end 1455b. The capsule 1422 may be rigid and made of metal. However, unlike the capsule 522, the capsule 1422 includes a raised lip 1468 at the proximal end 1455b. When everted and in the delivery configuration, the first end 118 of the brim 115 can be tucked under the raised lip 1468 of the capsule 1422 so that only the first end 118 of the brim 115 and the capsule 1422 are overlapping along the raised lip 1468.

The raised lip 1468 is flared or positioned radially outward relative to the remaining length of the capsule 1422. The flaring provided by the raised lip 1468 of the capsule 1422 reduces any localized stress that may be present on the frame 102 at the point where the brim 115 tucks under the raised lip 1468. As best shown in FIG. 15, the raised lip 1468 has a longitudinal length L1 of about 2 mm and a height H1 of about 0.25 mm. H1 may be defined, for example, as a maximum depth that the flange extends from an inner surface 1423 of the capsule 1422. The capsule 1422 includes a first outer diameter D1 along a length thereof except along the raised lip 1468. Along the raised lip, the capsule 1422 includes a second outer diameter D2 which is greater than D1.

As described above, the brim 115 includes brim supports 116 (sinusoidal wire forms) disposed within an integral folded pocket or hem of graft material 103B. When the brim 115 is tucked under the proximal end 1455B of the capsule 1422, the brim supports 116 help to maintain the everted position of the brim 115 during delivery. When everted and in the delivery configuration, and when tucked under the distal capsule 1422, the brim supports 116 are biased to move radially outward from the everted position and thus causes the brim 115 to press against an inner surface 1423 of the capsule 1422. The bias of the brim support 116 causes the brim 115 to remain disposed within the proximal end 1455a of the capsule 1422 during delivery through the vasculature.

As stated above, the brim 115 of the prosthetic heart valve 100 may have alternative configurations from the one depicted in FIGS. 1-4. For example, rather than forming an integral folded pocket as described above, the brim 115 may include a single layer of fabric material to reduce the overall profile of the brim 115. In any embodiment hereof, the brim 115 can include a biocompatible echogenic coating (not shown) on the graft material 103B to provide echogenicity to the brim 115. The coating can optionally be bioresorbable. It is further envisioned that a microbubble trap of any of the types known for providing enhanced ultrasound imaging could be incorporated into the brim 115.

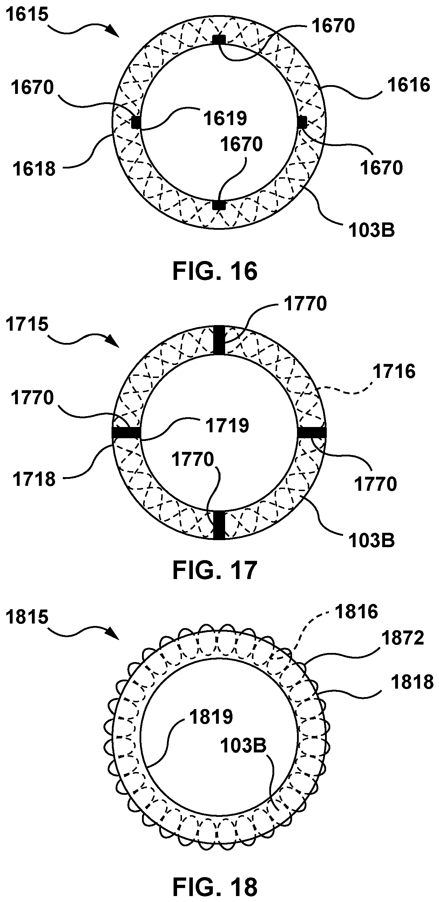

FIG. 16 depicts a brim 1615 according to another embodiment hereof which may be utilized within the prosthetic heart valve 100. The brim 1615 can be identically configured as the brim 115 and used the same except as explicitly stated. Similar to the brim 115, the brim 1615 is formed by a brim support 1616 and a portion of graft material 103B that extends past or beyond the inflow end of the anchor 102B. The graft material 103B includes an integral folded pocket or hem beyond the inflow end of the anchoring member 102B, and the brim support 1616 is disposed within this folded pocket of the graft material 103B. In the depicted embodiment, the brim support 1616 includes overlapping, 180 degree out of phase sinusoidal wire forms. The brim 1615 includes a first end or edge 1618 and a second end or edge 1619. To improve navigability, the brim 1615 includes one or more echogenic ribs and/or markers 1670. The ribs or markers 1670 are echogenic to enable TEE (trans-esophageal echocardiography) navigability of a delivery system 520 including the prosthetic heart valve 100 in the delivery configuration thereon. The plurality of ribs 1670 may approximately equally spaced or positioned around a circumference of the brim 1615. Each rib 1670 is disposed adjacent to the second end 1619 of the brim 1615. The ribs 1670 are disposed on or attached to the surface of the brim 1615 that is exterior when the brim 1615 is everted so that the ribs 1670 are exposed and visible when positioned over the anchoring member 102B. In an embodiment, the ribs 1670 may be made of a shape memory material (e.g., nitinol) and be configured to assist in reverting the brim 1615 after release from the capsule 522. In other words, the ribs 1670 may be biased to position the brim 1615 in the reverted position or arrangement of FIG. 1.

FIG. 17 depicts a brim 1715 according to another embodiment hereof which may be utilized within the prosthetic heart valve 100. The brim 1715 can be identically configured as the brim 115 and used the same except as explicitly stated. Similar to the brim 115, the brim 1715 is formed by a brim support 1716 and a portion of graft material 103B that extends past or beyond the inflow end of the anchor 102B. The graft material 103B includes an integral folded pocket or hem beyond the inflow end of the anchoring member 102B, and the brim support 1716 is disposed within this folded pocket of the graft material 103B. In the depicted embodiment, the brim support 1716 includes overlapping, 180 degree out of phase sinusoidal wire forms. The brim 1715 includes a first end or edge 1718 and a second end or edge 1719. To improve navigability, the brim 1715 includes one or more echogenic ribs and/or markers 1770. The ribs or markers 1770 are echogenic to enable TEE (trans-esophageal echocardiography) navigability of a delivery system 520 including the prosthetic heart valve 100 in the delivery configuration thereon. The plurality of ribs 1770 may approximately equally spaced or positioned around a circumference of the brim 1715. Each rib 1770 may extend along a length of the brim 1715 from and/or between the first end 1718 and the second end 1719 thereof. The ribs 1770 are disposed on or attached to the surface of the brim 1715 that is exterior when the brim 1715 is everted so that the ribs 1770 are exposed and visible when positioned over the anchoring member 102B. In an embodiment, the ribs 1770 may be made of a shape memory material (e.g., nitinol) and be configured to assist in reverting the brim 1715 after release from the capsule 522. In other words, the ribs 1770 may be biased to position the brim 1715 in the reverted position or arrangement of FIG. 1.

FIG. 18 depicts a brim 1815 according to another embodiment hereof which may be utilized within the prosthetic heart valve 100. The brim 1815 can be identically configured as the brim 115 and used the same except as explicitly stated. Similar to the brim 115, the brim 1815 is formed by a brim support 1816 and a portion of graft material 103B that extends past or beyond the inflow end of the anchor 102B. In this embodiment, the graft material 103B is a single layer of material and does not include an integral folded pocket or hem. In the depicted embodiment, the brim support 1816 includes overlapping, 180 degree out of phase sinusoidal wire forms. The brim 1815 includes a first end or edge 1818 and a second end or edge 1819. The brim support 1816 of this embodiment is configured to extend past the first end 1818 of the graft material 103B. The portion of the brim support 1816 that extends past the first end 1818 of the graft material 103B is herein referred to as exposed peaks 1872. When everted and in the delivery configuration, and when tucked into the distal capsule 522 or the distal capsule 1422, the exposed peaks 1872 of the brim support 1816 extends within the capsule 522, 1422 to assist in maintaining the brim 1815 within the capsule 522, 1422. In one example, the brim support 1816 extends past the first end 1818 distance of at least 2 mm.

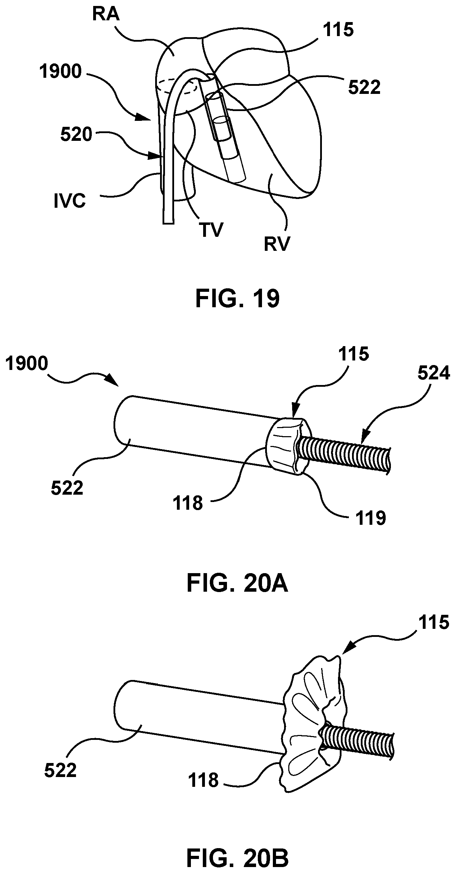

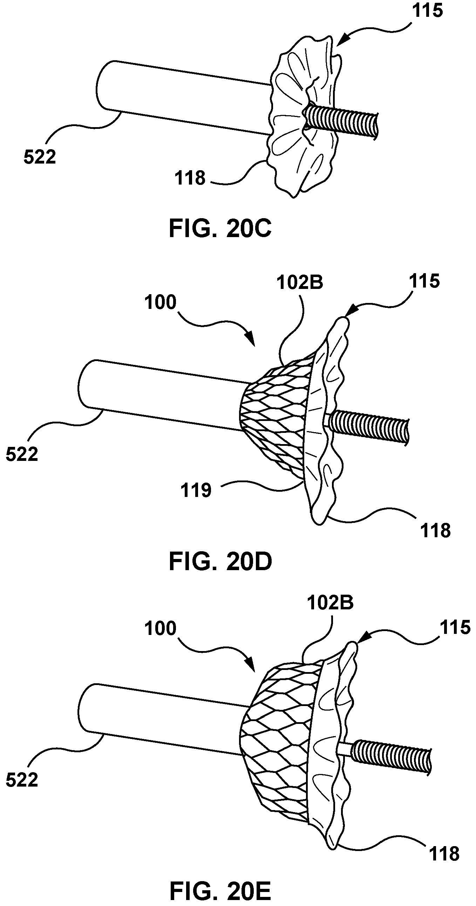

FIGS. 19-20E schematically illustrate one non-limiting method of the present disclosure. In various methods of the present disclosure, a system 1900 is prepared by crimping and loading the prosthetic heart valve 100 onto the piston 554 of the delivery system 520. The capsule 522 is positioned over the outflow portion of the prosthetic heart valve 100 and the brim 115 (or an alternate brim of the present disclosure) is everted over the uncovered portion 1162 of the frame 102 so that the capsule 522 and the brim 115 collectively sheathe and entirely cover the anchoring member 102B, as described above with respect to FIGS. 11 and 12. Once system 1900 is in delivery configuration of FIGS. 19 and 20A, for example (see also, FIG. 12), the system 1900 is directed to a target site via transcatheter procedure. In an example, the target site is a native heart valve or previous implanted prosthesis heart valve. In another example, the target site may be a mitral valve via the left atrium. In the illustrated example, the target site is at a tricuspid valve TV. In the example of FIGS. 19-20E, the system 1900 is directed through an inferior vena cava IVC into the right atrium RA and the capsule 522 is directed down though the tricuspid valve TV annulus at least partially within the right ventricle RV. In some examples, the brim 115 does not extend into the right ventricle RV, or does not extend below the native valve annulus, and remains in the right atrium RA as the capsule 522 is distally advanced. In various embodiments, the system 1900 is inserted no more than 63 mm into the right ventricle RV.

After the capsule 522 and the prosthetic heart valve 100 are positioned as desired *in situ,* deployment of the prosthetic heart valve 100 may be initiated via reversion of the brim 115. In embodiments including the brim lock 564, the brim lock 564 can be released at this stage to allow the brim 115 to at least partially revert at the desired time. If the brim 115 is tucked into the proximal end 555b of the capsule 522, the capsule 522 may be slightly distally advanced (via hydraulics, as described above with respect to FIGS. 8-10) in order to release the brim 115, which may automatically revert upon release from the capsule 522. Reversion of the brim 115 can be aided by the incorporation of echogenic ribs 1670, 1770 (which may be formed from a shape memory material) provided on the brim 115 as discussed above. As the brim 115 reverts, a sonographer may visualize the first end 118 of the brim 115 to visualize and understand a location of the brim 115 inflection at the second end 119. Although reversion of the brim 115 may be aided by echogenic ribs 1670, 1770, the brim support 116 of the brim 115 alone is sufficient to cause the brim 115 to revert.

During or after reversion of the brim 115, the frame 102 of the prosthetic heart valve 100 is then deployed via distal advancement of the capsule 522 as described above with respect to FIGS. 8-10. As described above, hydraulics are used to deploy the capsule 522 distally and away from the prosthetic heart valve 100. At the stage of FIG. 20D, the brim 115 will continuously expand and revert toward an expanded configuration within the right atrium as the frame 102 is released, unfolding proximally to a point where the brim 115 is approximately perpendicular to the longitudinal axis of the capsule 522. At this point the sonographer may readily view the inflection point at an apex (near the second end 119) of an angle between the anchoring member 102B and the brim 115. It is noted that at the stages of FIG. 20C-D the sonographer may visualize the inflection point using the echogenic band 106 (not shown on the schematic views of FIGS. 20C-D) to guide the brim inflection point to the valve annulus (e.g., mitral or tricuspid valve annulus). With the brim 115 reverted and the anchoring member 102B beginning to expand, depth checks can be performed. While the echogenic band 106 may aid in visualization, the brim inflection point between the anchoring member 102B and the brim 115 is sufficient to visualize using echo. The inflection point of the brim 115 may then be adjusted to align closely with the native valve annulus.

Further distal advancement of the capsule 522 eventually uncovers or unsheathes the prosthetic heart valve 100, such that the prosthetic heart valve 100 decouples from the piston 554 and radially expands towards the expanded configuration of FIG. 1. In any method of the disclosure, once the prosthetic heart valve 100 is fully deployed and released from the delivery system 520, the capsule 522 can be proximally withdrawn through the prosthetic heart valve 100 and withdrawn from the patient in the same manner that the delivery system 520 was delivered.

During prototyping and experimentation with various embodiments of the disclosure, the present inventors observed that the embodiments of the disclosure including an everted brim and shortened capsule will match or exceed the visibility of the brim inflection point under echogenic guidance, leading to improved ability to guide the placement of the prosthetic heart valve in the native annulus. For example, at the stage of FIG. 20A, due to the distally folded or everted brim 115, the brim 115 immediately "flowers" outward or reverts to a position that is perpendicular (90 degrees) with respect to the longitudinal axis of the capsule 522 (FIG. 20C). Compared to a system that fully sheathes the prosthetic heart valve with a capsule, initial flowering occurs with the brim 115 starting from an in-line configuration with respect to the anchoring member 102B, which results in a muted inflection angle and reduced visibility of the inflection point on echocardiography. This significantly reduces the ability to correctly align the prosthetic heart valve with the native annulus.

System insertion depth into a ventricle may further vary as indicted in the Table 1 below. All units of Table 1 are in millimeters, and include a tolerance of 4 mm.

**TABLE 1**

| Valve Diameter | 42 | 48 | 54 |
|---|---|---|---|
| Ventricular insertion depth (design with reverted brim) | 62 | 69 | 75 |
| Adjustment for everted brim shortening the capsule | -8 | -8 | -8 |
| Adjusted delivery system insertion depth (everted brim) | 54 | 61 | 67 |
| If the everted brim is found to reduce the capsule length by 10 mm | -10 | -10 | -10 |
| Adjusted delivery system insertion depth (everted brim) | 52 | 59 | 65 |

During valve deployment described above, it may become necessary to reposition, recover, recapture, or retrieve a partially deployed prosthetic heart valve 100. Partially deployed, as used herein, refers to a delivery state of the prosthetic heart valve 100 in which the capsule 522 has been distally advanced such that at least a portion of the frame 102 has been deployed, while the attachment bars 112 are still coupled to the piston 554 with the capsule 522 disposed thereover. Bailout procedures may become necessary after failed valve deployment when the prosthetic heart valve 100 is mislocated or damaged during deployment. Referring now in addition to FIGS. 21A-21D, in the event recapture of the prosthetic heart valve 100 is desired during the deployment thereof, the delivery system 520 can optionally be provided with a brim recapture member 2180. The brim recapture member 2180 is a flexible elongated component such as a suture, wire, cord or filament. With reference to FIG. 21A, the brim recapture member 2180 may be pre-loaded into the brim 115 around a circumference of the brim 115 via weaving or otherwise attaching the brim recapture member 2180 to the brim support 116. The brim recapture member 2180 has a sufficient length to permit full expansion of the brim 115, but may be utilized to recapture the brim 115 if necessary. In one example, the brim recapture member 2180 is positioned midway between first and second ends or edges 118, 119 of the brim 115 to avoid being tucked into the capsule 522 when everted and in the delivery configuration. During loading of the prosthetic heart valve 100, prior to delivery, a clinician will connect the brim recapture member 2180 to an elongated control member 2182 (which may also be a suture, wire, cord or filament) by passing a free end of the control member 2182 through the brim recapture member 2180 as shown in FIG. 21B. The control member 2182 extends from the brim 115 to the handle 527 of the inner steerable catheter 526, and a proximal end thereof (not shown) is attached to an actuator (not shown) on the handle 527 that is accessible to a user. The control member 2182 may extend within the inner steerable catheter 526 via a port 2184.

FIGS. 21C-21D illustrate recapture of the prosthetic heart valve 100 via the brim recapture member 2180. As described above, the capsule 522 may be retracted proximally via tension cable 530 and recapture piston 590 in order to recapture the frame 102. FIG. 21C illustrates the stage in which the capsule 522 has been retracted proximally to recapture the frame 102 but the brim 115 is deployed or partially deployed. At this stage, as shown in FIG. 21D, the operator can proximally tension the control member 2182 via the actuator on the handle 527 to result in tension applied to the brim recapture member 2180. Tension applied to the brim recapture member 2180 causes the deployed brim 115 to resume its everted or delivery configuration, allowing for bailout of the deployment process. More particularly, the second end or edge 119 of the brim 115 acts as an inflection point or hinge. In an embodiment, the tension control member 2182 may pass through the inflection point and then extend towards and around brim recapture member 2180. When tension is applied to the tension control member 2182, a moment is created around the inflection point (the second end or edge 119 of the brim 115), causing the brim 115 to evert. In addition, when tension is applied to the tension control member 2182, the brim recapture member 2180 radially compresses the brim 115.

To further reduce the profile of the everted brim, in another embodiment hereof (not shown), the delivery system may include additional brim recapture member(s) and respective tension control member(s). For example, in addition to the brim recapture member 2180 and associated tension control member 2182, the delivery system may include an additional or second brim recapture member adjacent to the first end or edge 118 of the brim 115 and an additional or second tension control member for applying tension to the second brim recapture member.

Once prosthetic heart valve 100 is fully deployed and released from the capsule 522, the control member 2182 can be disconnected from the brim recapture member 2180 to disconnect or decouple the prosthetic heart valve 100 from the delivery system 520. In one embodiment, the control member 2182 is a suture that can be pulled from the delivery system 520 to disengage with the brim recapture member 2180. More particularly, the control member 2182 may extend through the inner steerable catheter 526, loop around the brim recapture member 2180, and extend back through the inner steerable catheter 526. The control member 2182 is removed from the brim recapture member 2180 by pulling on one end of thereof until the entire control member 2182 is pulled through and removed from the delivery system 520. The brim recapture member 2180 remains disposed around the brim 115, on the deployed prosthetic heart valve 100.

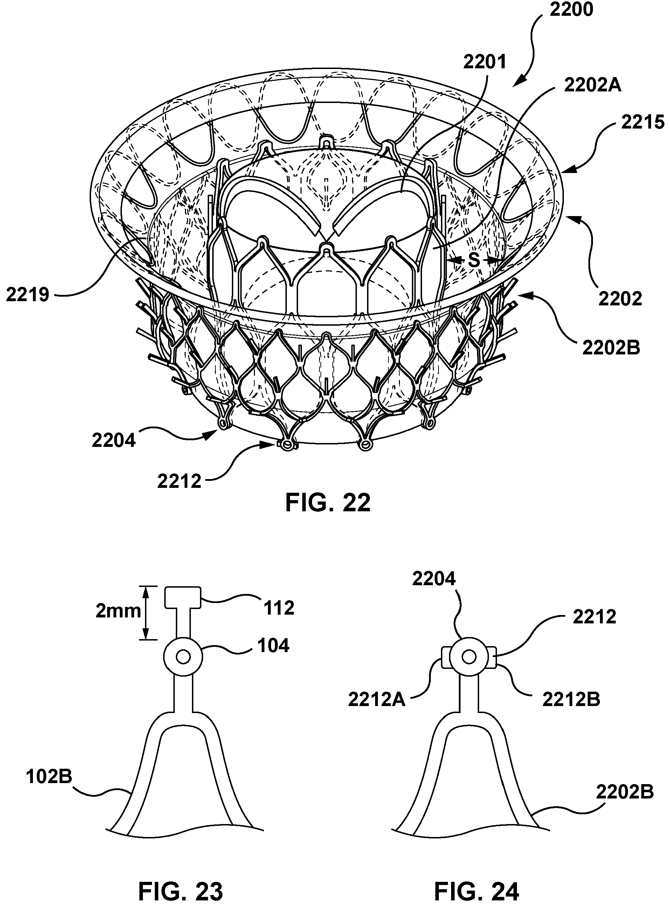

Another aspect of the present disclosure will now be described with respect to FIGS. 22-24. To further reduce the deployment depth of the system 1900 through an annulus, such as a valve annulus, any prosthetic heart valves of the disclosure (e.g., prosthetic heart valve 100 including any eversible brim described herein) can be modified so that the attachment bars 112 at the outflow end 109 of the valve support 102A can extend from one or more connector components 104 at the outflow end 109 of the valve support 102A.

More particularly, a prosthetic heart valve 2200 according to another embodiment is depicted in FIG. 22. The prosthetic heart valve 2200 can be the same as prosthetic heart valve 100 and used the same except as explicitly stated. As described above with respect to the prosthetic heart valve 100, the prosthetic heart valve 2200 includes a frame 2202 and a valve component 2201 disposed therein. The frame 2202 of the prosthetic heart valve 2200 includes a valve support 2202A at least partially surrounded by and attached to an anchoring member 2202B. The valve support 2202A is configured to support the valve component 2201 therein. The prosthetic heart valve 2200 also includes a brim 2215, which is the same as the brim 115, and functions to evert and sheathe or cover a portion of the frame 2202 during delivery. At the outflow end thereof, the valve support 2202A is attached to the anchoring member 2202B via a plurality of connector components 2204. In an embodiment, the plurality of connector components 2204 are rivets. In addition, at the outflow end thereof, the valve support 2202A includes a plurality of attachment bars 2212 that function to releasably couple the prosthetic heart valve 2200 to a delivery system. Unlike the prosthetic heart valve 100, the attachment bars 2212 are formed on one or more connector components 2204 rather than extending therefrom. Stated another way, a distance from a second end 2219 of the brim 2215 to the attachment bar(s) 2212 does not exceed a distance from the second end 2219 of brim 2215 to the connector component(s) 2204 to which the attachment bars 112 are attached.

More particularly, the structural change of the attachment bars 2212 may be best shown by a comparison of FIGS. 23 and 24. FIG. 23 illustrates a schematic view of an attachment bar 112 of the prosthetic heart valve 100 of FIG. 1, while FIG. 24 illustrates a schematic view of an attachment bar 2212 of the prosthetic heart valve 2200 of FIG. 22. In FIG. 23, the attachment bar 112 is generally a T-shape and extends in an axial direction from a connector component 104. The length of the attachment bar 112 is approximately 2 mm. Conversely, in FIG. 24, the attachment bar 2212 is formed on or overlaps with the connector component 2204. The attachment bar 2212 includes opposing wings or tabs 2212A, 2212B that extend in a circumferential direction from the connector component 2204. The attachment bar 2212 does not extend in an axial direction from the connector component 2204, and thus the overall length of the prosthetic heart valve 2200 (having the attachment bars 2212 thereon) is relatively shorter than the overall length of the prosthetic heart valve 100 (having the attachment bars 112 thereon). Particularly, the prosthetic heart valve 2200 is approximately 2 mm shorter than the prosthetic heart valve 100 (i.e., the length of the attachment bars 112). The attachment bars 2212 may still function similarly to the attachment bars 112, and are configured to be received within the grooves 558 of the piston 554 for coupling thereto.

The practical result of the configuration of the attachment bars 2212 is that the length of the piston mount 524B can be reduced by the same amount as the length reduction described above (about 2 mm in some examples). As the piston mount 524B is typically rigid, the length reduction provides greater flexibility to a distal end of the delivery system 520 proximate the capsule 522. Particularly, the relatively shorter piston mount 524B improves the ability of the distal portion of the delivery system 520 to navigate around a tighter radius. Generally, the piston mount 524B is at least as long as the compressed length of the frame 102 and the piston 554 (which is mounted over a distal end of the piston mount 524). Since the length of the coupling between the piston 554 and the prosthetic heart valve 2200 is reduced due to the configuration of the attachment bars 2212, the length of the piston mount 524B may be similarly reduced. In addition to a reduction in length of the piston mount 524B, the attachment bars 2212 also result in a reduction of length of the capsule 522 due to the reduction in length of the prosthetic heart valve 2200. Further shortening of the capsule also improves the ability of the distal portion of the delivery system to navigate around a tighter radius and allows the delivery system to be configured to treater relatively shorter right ventricles and therefore a higher number of patients.

Although embodiments hereof are described with exemplary prosthetic heart valves and an exemplary delivery system, aspects of the present disclosure are not intended to be limited to the examples described herein. For example, although depicted on the prosthetic heart valve 2200 having the brim 2215 which functions to evert and sheathe or cover a portion of the frame 102 during delivery, the attachment bars 2212 may be utilized on any prosthetic heart valve that couples to a piston of a delivery system. The inclusion of the brim 2215 is not required. Use of the attachment bars 2212 on any prosthetic heart valve that couples to a piston of a delivery system results in a relatively shorter piston mount to thereby improves the ability of the distal portion of the delivery system to navigate around a tighter radius.

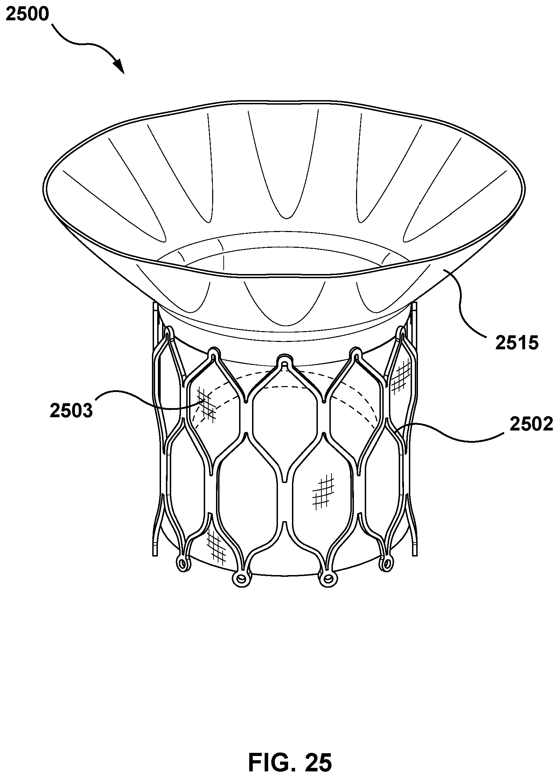

In addition, although the brim 115 is described herein with respect to a prosthetic heart valve having inner and outer frames, the present disclosure may be applied to any prosthetic heart valve having an eversible brim and at least one frame. For example, FIG. 25 depicts another prosthetic heart valve 2500 including a frame 2502 and a brim 2515 which functions to evert and sheathe or cover a portion of the frame 2502 during delivery. The prosthetic heart valve 2500 may include a valve component (not shown), similar to the valve component 101 of the prosthetic heart valve 100, disposed therein. The frame 2502 includes a skirt or graft material 2503 secured thereto. More particularly, the graft material 2503 is coupled to an inner surface of the frame 2502 to line a portion thereof. The graft material 2503 may be the same as any materials described herein for the graft material 103A, 103B.

The brim 2515 that extends outwardly from a first or inflow end of the frame 2502. The brim 2515 may be similar to the brim 115, and include a brim support and a flexible web, which in this embodiment is a portion of graft material that extends past or beyond the inflow end of the frame 2502. The brim 2515 may act as an atrial retainer, if present, and to serve such a function the brim 2515 may be configured to engage tissue above a native annulus, such as a supra-annular surface or some other tissue in the right atrium, to thereby inhibit downstream migration of a prosthetic heart valve 2500. Accordingly, the brim 2515 is of a larger expanded diameter than the frame 2502 and extends radially outward from the frame 2502. The portion of graft material connecting the brim 2515 to the frame 2502 is referred to herein as a brim hinge that is configured to permit the brim 2515 to hinge and/or flex with respect to the remainder of the prosthetic heart valve 2500. Thus, the brim 2515 is a floppy structure that can readily flex with respect to the frame 2502. In some embodiments, one or more components of the brim 2515 can be made of or include a radiopaque or echogenic material that enables TEE (trans-esophageal echocardiography) tracking of the prosthetic heart valve 2500, and particularly tracking of the movement of the brim 2515, *in situ.* Similar to the brim 115, the brim 2515 is configured to distally fold or evert in order to cover at least a portion of the frame 2502 during delivery thereof. Thus, during delivery within a vasculature, a portion of the prosthetic heart valve 2500 is used to sheathe a portion of the frame 2502. More particularly, the brim 2515 of the prosthetic heart valve 2500 is everted so that both the brim 2515 and a capsule (such as capsule 522) collectively sheathe the frame 2502 to a degree such that the frame 2502 is not substantially exposed. Utilizing the brim 25 15 to sheathe a portion of the frame 2502 during delivery allows for the length of the capsule to be reduced, while still sheathing the frame 2502 to provide atraumatic delivery.

In addition, although described herein with respect to a delivery system having inner and outer steerable catheters, and other features, the present disclosure may be applied to any delivery system having a capsule for radially compressing a prosthetic heart valve, with the capsule having a length that is shorter than a length of the prosthetic heart valve. As another example, aspects of the present disclosure are not intended to be limited to delivery systems having capsules that are hydraulically controlled.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A prosthesis delivery system (520) comprising:
a tubular capsule (522); and
a prosthesis (100) having a brim (115), the brim having a first end (118), wherein the prosthesis delivery system has a delivery configuration for delivery within a vasculature, in the delivery configuration a portion of the prosthesis is disposed in the capsule and at least a portion of the brim is outside the capsule and everted to extend toward the capsule prior to deployment of the prosthesis from the capsule.

2. The prosthesis delivery system of claim 1, wherein the tubular capsule has a closed end and an open end, in particular, the tubular capsule further including a piston positioned within the capsule, in particular, wherein said closed end and said piston form at least in part a chamber, in particular, further including a fluid source coupled to said chamber.

3. The prosthesis delivery system of claim 1, wherein the first end of the brim is releasably coupled to the capsule, in particular, wherein the brim is releasably coupled to the capsule with sutures.

4. The prosthesis delivery system of claim 1, wherein the prosthesis delivery system has a partially deployed configuration where the brim has been released from the capsule.

5. The prosthesis delivery system of claim 1, wherein the brim is configured to move toward a reverted position when released.

6. The prosthesis delivery system of claim 1, wherein the first end of the brim is tucked inside the capsule, or
wherein the capsule has an open end with a raised lip, first end of the brim being tucked in the raised lip.

7. The prosthesis delivery system of claim 1, wherein the prosthesis has a plurality of fixation elements, the everted brim covering the plurality of fixation elements.

8. The prosthesis delivery system of claim 1, wherein the prosthesis is a self-expanding prosthesis configured to expand from a compressed configuration toward an expanded configuration.

9. The prosthesis delivery system of claim 1, wherein the prosthesis is a prosthetic heart valve.

10. The prosthesis delivery system of claim 1, wherein said prosthesis includes a frame (102), said frame being coupled to said brim, in particular,
wherein said brim extends from said frame, and/or
wherein at least a portion of said frame extends out from the capsule, in particular,
wherein said brim and said capsule collectively entirely cover said frame.

11. The prosthesis delivery system of claim 1, wherein the capsule is configured to distally advance to unsheathe the prosthesis.

12. A method of loading a prosthesis (100) into a delivery capsule comprising:
positioning a prosthesis, having a support structure and a brim (115) extending from the support structure, into a tubular capsule (522) such that a portion of the support structure extends from the capsule; and
everting the brim such that the brim and the tubular capsule cover the support structure.

13. The method of claim 12, wherein the prosthesis is everted such that the prosthesis and capsule completely cover the prosthesis support structure, or further including tucking a portion of the prosthesis into the capsule.

14. The method of claim 13, further including a valve supported in the prosthesis support structure, in particular,
wherein said support structure comprises a frame (102), in particular,
wherein the support structure comprises an inner frame and an outer frame with the inner frame positioned in the outer frame.

## Patentansprüche

1. Protheseneinführsystem (520), umfassend:
eine rohrförmige Kapsel (522); und
eine Prothese (100), die einen Rand (115) aufweist, wobei der Rand ein erstes Ende (118) aufweist, wobei das Protheseneinführsystem eine Einführkonfiguration für eine Einführung innerhalb eines Gefäßsystems aufweist, wobei in der Einführkonfiguration ein Abschnitt der Prothese in der Kapsel angeordnet ist und mindestens ein Abschnitt des Randes sich außerhalb der Kapsel befindet und umgestülpt ist, um sich vor dem Ausgeben der Prothese aus der Kapsel zu der Kapsel hin zu erstrecken.

2. Protheseneinführsystem nach Anspruch 1, wobei die rohrförmige Kapsel ein geschlossenes Ende und ein offenes Ende aufweist, insbesondere die rohrförmige Kapsel ferner einen innerhalb der Kapsel positionierten Kolben einschließt, insbesondere wobei das geschlossene Ende und der Kolben mindestens teilweise eine Kammer ausbilden, insbesondere ferner eine Fluidquelle einschließen, die an die Kammer gekoppelt ist.

3. Protheseneinführsystem nach Anspruch 1, wobei das erste Ende des Randes lösbar an die Kapsel gekoppelt ist, insbesondere, wobei der Rand mit Nähten lösbar an die Kapsel gekoppelt ist.

4. Protheseneinführsystem nach Anspruch 1, wobei das Protheseneinführsystem eine teilweise abgegebene Konfiguration aufweist, in der der Rand aus der Kapsel gelöst wurde.

5. Protheseneinführsystem nach Anspruch 1, wobei der Rand konfiguriert ist, um sich zu einer zurückgesetzten Position hin zu bewegen, wenn gelöst.

6. Protheseneinführsystem nach Anspruch 1, wobei das erste Ende des Randes in die Kapsel eingesteckt ist, oder
wobei die Kapsel ein offenes Ende mit einer erhabenen Lippe aufweist, wobei ein erstes Ende des Randes in die erhabene Lippe eingesteckt ist.

7. Protheseneinführsystem nach Anspruch 1, wobei die Prothese eine Vielzahl von Fixierungselementen aufweist, wobei der umgestülpte Rand die Vielzahl von Fixierungselementen bedeckt.

8. Protheseneinführsystem nach Anspruch 1, wobei die Prothese eine selbstexpandierende Prothese ist, die konfiguriert ist, um von einer komprimierten Konfiguration zu einer expandierten Konfiguration hin zu expandieren.

9. Protheseneinführsystem nach Anspruch 1, wobei die Prothese eine Herzklappenprothese ist.

10. Protheseneinführsystem nach Anspruch 1, wobei die Prothese einen Rahmen (102) einschließt, wobei der Rahmen an den Rand gekoppelt ist, insbesondere,
wobei sich der Rand von dem Rahmen erstreckt, und/oder
wobei sich mindestens ein Abschnitt des Rahmens aus der Kapsel heraus erstreckt, insbesondere,
wobei der Rand und die Kapsel gemeinsam den Rahmen vollständig bedecken.

11. Protheseneinführsystem nach Anspruch 1, wobei die Kapsel konfiguriert ist, um sich distal vorzubewegen, um die Prothese zu enthüllen.

12. Verfahren zum Beladen einer Prothese (100) in eine Einführkapsel, umfassend:
derartiges Positionieren einer Prothese, die eine Stützstruktur und einen Rand (115) aufweist, der sich von der Stützstruktur erstreckt, in eine rohrförmige Kapsel (522), dass sich ein Abschnitt der Stützstruktur aus der Kapsel erstreckt; und
derartiges Umstülpen des Randes, dass der Rand und die rohrförmige Kapsel die Stützstruktur bedecken.

13. Verfahren nach Anspruch 12, wobei die Prothese derart umgestülpt wird, dass die Prothese und die Kapsel die Prothesenstützstruktur vollständig bedecken, oder ferner das Einstecken eines Abschnitts der Prothese in die Kapsel einschließt.

14. Verfahren nach Anspruch 13, ferner umfassend ein Ventil, das in der Prothesenstützstruktur gestützt ist, insbesondere,
wobei die Stützstruktur einen Rahmen (102) umfasst, insbesondere,
wobei die Stützstruktur einen Innenrahmen und einen Außenrahmen umfasst, wobei der Innenrahmen in dem Außenrahmen positioniert ist.

## Revendications

1. Système de pose de prothèse (520) comprenant :
une capsule tubulaire (522) ; et
une prothèse (100) ayant un bord (115), le bord ayant une première extrémité (118), dans lequel le système de pose de prothèse a une configuration de pose pour une pose au sein d'un système vasculaire, dans la configuration de pose une partie de la prothèse est disposée dans la capsule et au moins une partie du bord est à l'extérieur de la capsule et retournée pour s'étendre vers la capsule avant le déploiement de la prothèse à partir de la capsule.

2. Système de pose de prothèse selon la revendication 1, dans lequel la capsule tubulaire a une extrémité fermée et une extrémité ouverte, en particulier, la capsule tubulaire comportant en outre un piston positionné au sein de la capsule, en particulier, dans lequel ladite extrémité fermée et ledit piston forment au moins en partie une chambre, en particulier, comportant en outre une source de fluide accouplée à ladite chambre.

3. Système de pose de prothèse selon la revendication 1, dans lequel la première extrémité du bord est accouplée de manière libérable à la capsule, en particulier, dans lequel le bord est accouplé de manière libérable à la capsule avec des sutures.

4. Système de pose de prothèse selon la revendication 1, dans lequel le système de pose de prothèse a une configuration partiellement déployée où le bord a été libéré de la capsule.

5. Système de pose de prothèse selon la revendication 1, dans lequel le bord est configuré pour se déplacer vers une position retournée lorsqu'il est libéré.

6. Système de pose de prothèse selon la revendication 1, dans lequel la première extrémité du bord est repliée à l'intérieur de la capsule, ou
dans lequel la capsule a une extrémité ouverte avec une lèvre surélevée, une première extrémité du bord étant repliée dans la lèvre surélevée.

7. Système de pose de prothèse selon la revendication 1, dans lequel la prothèse a une pluralité d'éléments de fixation, le bord retourné recouvrant la pluralité d'éléments de fixation.

8. Système de pose de prothèse selon la revendication 1, dans lequel la prothèse est une prothèse à auto-élargissement configurée pour s'élargir d'une configuration comprimée à une configuration élargie.

9. Système de pose de prothèse selon la revendication 1, dans lequel la prothèse est une valve cardiaque prothétique.

10. Système de pose de prothèse selon la revendication 1, dans lequel ladite prothèse comporte un cadre (102), ledit cadre étant accouplé audit bord, en particulier,
dans lequel ledit bord s'étend à partir dudit cadre, et/ou
dans lequel au moins une partie dudit cadre s'étend à l'extérieur de la capsule, en particulier,
dans lequel ledit bord et ladite capsule recouvrent collectivement et entièrement ledit cadre.

11. Système de pose de prothèse selon la revendication 1, dans lequel la capsule est configurée pour avancer de manière distale pour dégainer la prothèse.

12. Procédé de chargement d'une prothèse (100) dans une capsule de pose comprenant :
le positionnement d'une prothèse, ayant une structure de support et un bord (115) s'étendant à partir de la structure de support, dans une capsule tubulaire (522) de telle sorte qu'une partie de la structure de support s'étende à partir de la capsule ; et
le retournement du bord de telle sorte que le bord et la capsule tubulaire recouvrent la structure de support.

13. Procédé selon la revendication 12, dans lequel la prothèse est retournée de telle sorte que la prothèse et la capsule recouvrent complètement la structure de support de prothèse, ou comportant en outre le repli d'une partie de la prothèse dans la capsule.

14. Procédé selon la revendication 13, comportant en outre une valve supportée dans la structure de support de prothèse, en particulier,
dans lequel ladite structure de support comprend un cadre (102), en particulier,
dans lequel la structure de support comprend un cadre interne et un cadre externe avec le cadre interne positionné dans le cadre externe.
